(19) Europäisches Patentamt
European Patent Office
Office européen des brevets

(11) Publication number: 0 317 336
A1

(12) # EUROPEAN PATENT APPLICATION

(21) Application number: 88310907.6

(22) Date of filing: 18.11.88

(51) Int. Cl.⁴: A 01 N 47/36
C 07 D 401/12

(30) Priority: 20.11.87 US 123481 01.08.88 US 226934
20.11.87 US 123482

(43) Date of publication of application:
24.05.89 Bulletin 89/21

(84) Designated Contracting States: ES GR

(71) Applicant: E.I. DU PONT DE NEMOURS AND COMPANY
1007 Market Street
Wilmington Delaware 19898 (US)

(72) Inventor: Wexler, Barry Arthur
2205 Patwynn Road
Wilmington Delaware 19810 (US)

Amuti, Kofi Sam
5412 Valley Green Drive
Wilmington Delaware 19808 (US)

Leep, Daniel Carl
12 Farmingdale Lane
Newark Delaware 19714 (US)

(74) Representative: Hildyard, Edward Martin et al
Frank B. Dehn & Co. European Patent Attorneys Imperial
House 15-19 Kingsway
London WC2B 6UZ (GB)

(54) **Herbicidal alkanoylpyridine sulfonylureas.**

(57) This invention relates to a method for controlling the growth of undesired vegetation in maize, soybeans and plantation crops, and in fallow applications by applying an effective amount of certain alkanoylpyridinesulfonylureas to the locus of the area to be protected.

EP 0 317 336 A1

Description

# HERBICIDAL ALKANOYLPYRIDINESULFONYLUREAS

## Related Applications

This is a continuation-in-part of U.S. Serial Nos. 07/123481, filed November 20, 1987 and U.S. Serial No. 07/123482, filed on November 20, 1987.

## Background of the Invention

This invention relates to certain herbicidal sulfonylurea compounds as a general or selective preemergent or postemergent herbicide or as a plant growth regulant. More specifically, this invention relates to a method of use of said compounds to control undesired vegetation in crop and fallow applications.

New compounds effective for controlling the growth of undesired vegetation are in constant demand. In the most common situation, such compounds are sought to selectively control the growth of weeds in useful crops such as cotton, rice, corn, wheat and soybeans, to name a few. Unchecked weed growth not only in such crops but also in plantation crops can cause significant losses, reducing profit to the farmer and increasing costs to the consumer. In other situations, herbicides are desired which will control all plant growth. Examples of areas in which complete control of all vegetation is desired are areas around railroad tracks, storage tanks and industrial storage areas. There are many products commercially available for these purposes, but the search continues for products which are more effective, less costly and environmentally safe.

The "sulfonylurea" herbicides are an extremely potent class of herbicides discovered within the last few years which generally consist of a sulfonylurea bridge, $-SO_2NHCONH-$, linking two aromatic or heteroaromatic rings.

U.S.S.N 93,416 discloses generically the compounds of the invention but does not specifically disclose said compounds of their usefulness in corn, soybeans, plantation crops and in fallow situations.

Japanese patent application 85/253,531, laid open May 22, 1987, discloses compounds of the formula

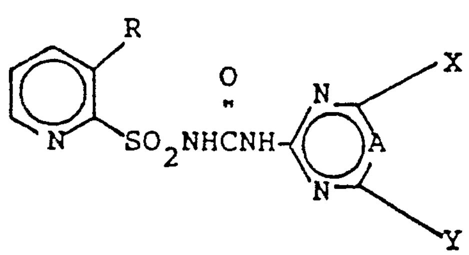

wherein
R is (in part) $COR_2$;
$R_2$ is $C_1$-$C_4$ alkyl;
X is methyl, methoxy or ethoxy; and
Y is halogen, methyl, methoxy or ethoxy; and
Z is N or CH.

This reference does not disclose the utility of the instant compounds in fallow land weed control or in the crop applications of the present invention.

U.S. 4,518,776 and EP-A-101,670 disclose, in part, a process for the preparation of compounds of the formula

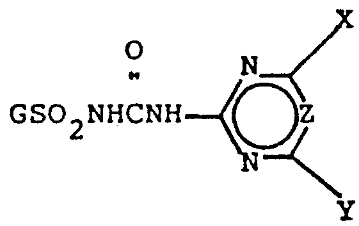

wherein

G is 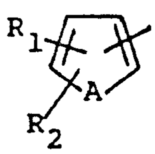 or 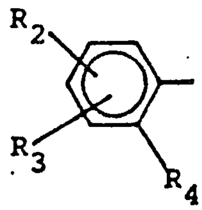 ;

$R_1$ is, among other values, CO-$C_1$-$C_4$-alkyl;
$R_2$ is H, halogen, $CF_3$, $NO_2$, $C_1$-$C_4$ alkyl or $C_1$-$C_4$ alkoxy;
A is O, S, $NR_5$, or -C=N-;
X is $C_1$-$C_4$ alkyl, $C_1$-$C_4$ haloalkyl, $C_1$-$C_4$ alkoxy, $C_1$-$C_4$ alkylthio, $C_1$-$C_4$ haloalkoxy, $C_1$-$C_4$ alkylamino or di-$C_1$-$C_4$ alkylamino;
Y is $C_1$-$C_4$ alkyl, $C_1$-$C_4$ alkoxy or $C_1$-$C_4$ haloalkoxy; and
Z is CH or N.

The references generically disclose the compounds of the invention, but do not specifically disclose such compounds or their use in the application of the present invention.

U.S. 4,521,597 and EP-A-107,624 disclose, in part, a process for the preparation of compounds of formula

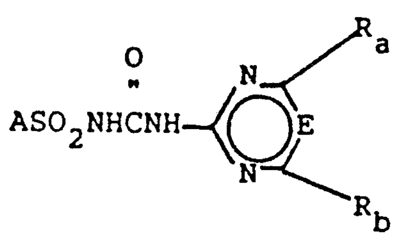

wherein

A is 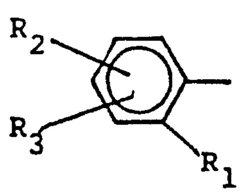 , 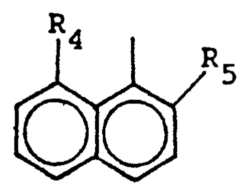 or

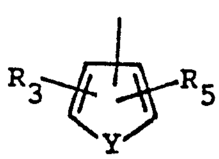 ;

$R_3$ is H, halogen, $NO_2$, $OCH_3$ or $CF_3$;
$R_5$ is, among others, -$COR_7$;
$R_7$ is H, $C_1$-$C_5$ alkyl, $C_1$-$C_5$ haloalkyl, $C_1$-$C_5$ alkoxy, $C_1$-$C_5$ haloalkoxy, $C_3$-$C_5$ alkenyloxy, $C_3$-$C_5$ alkynyloxy, phenoxy, benzyloxy, $C_1$-$C_5$ alkylthio or $NR_8R_9$;

Y is O, S, or $C(R_6)=N$;
$R_a$ is H, halogen, $C_1$-$C_5$ alkyl, $C_1$-$C_5$ haloalkyl, $C_1$-$C_5$ haloalkoxy, $C_1$-$C_5$ alkylthio, $C_2$-$C_{10}$ alkoxyalkyl or $C_2$-$C_{10}$ alkoxyalkoxy;
$R_b$ is the same as $R_a$ or $NR_cR_d$; and
E is CH or N.

These references generically disclose the compounds of the invention but not not specifically disclose compounds of the invention.

U.S. 4,549,898 discloses, in part, herbicidal sulfonylureas of the formula

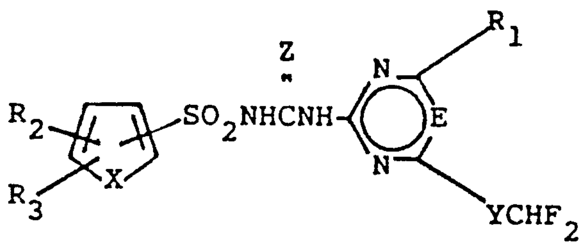

wherein
X is O, S, $NR_4$ or $C(R_5)=N$;
Y is O or S;
Z is O or S;
E is N or CH;
$R_1$ is H, $C_1$-$C_4$ alkyl, $C_1$-$C_4$ haloalkyl, $C_1$-$C_4$ haloalkoxy, $C_1$-$C_4$ alkoxy, halogen, $C_1$-$C_4$ alkylthio, $NR_6R_7$ or alkoxyalkyl containing not more than 4 carbon atoms;
$R_2$ is, among others, $C(W)R_8$;
$R_3$ is H, halogen, $C_1$-$C_3$ alkyl, $OCH_3$ or $CF_3$;
$R_8$ is H, $C_1$-$C_6$ alkyl, $C_1$-$C_4$ haloalkyl, $C_3$-$C_6$ cycloalkyl, $C_4$-$C_7$ cycloalkylalkyl or alkoxyalkyl containing not more than 4 carbon atoms; and
W is O or $=N$-O-$R_{10}$, wherein $R_{10}$ is hydrogen, $C_1$-$C_6$ alkyl or $C_3$-$C_6$ alkenyl.

Although compounds of the invention are generically disclosed, there is no specific disclosure of alkanoyl pyridines.

U.S. 4,435,206 discloses herbicidal pyridine-2-sulfonylureas, wherein the pyridine ring may be substituted by Cl, Br, F, $C_1$-$C_4$ alkyl, $C_1$-$C_4$ alkoxy, $C_1$-$C_4$ alkylthio, $NO_2$ or a carboxylic ester group, but does not disclose compounds of the invention.

## SUMMARY OF THE INVENTION

This invention pertains to the use of the compounds of Formula I as selective herbicides in maize (corn), soybeans and plantation crops and in fallow. Accordingly, this invention relates to a method for controlling undesired vegetation in crops comprising maize (corn), soybeans, plantation crops and on fallow land by applying to the locus to be protected a herbicidally effective amount of a compound of Formula I.

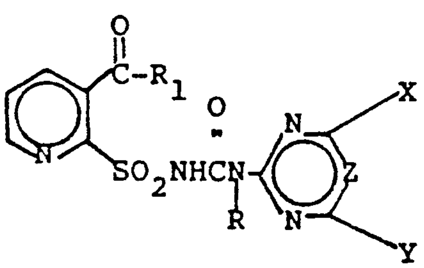

I

wherein
R is H or $CH_3$;
$R_1$ is $CH_3$, $CH_2CH_3$ or $(CH_2)_2CH_3$;
X is H, $C_1$-$C_2$ alkyl, $C_1$-$C_2$ alkoxy, $OCF_2H$, $OCH_2CF_2H$, $OCH_2CF_3$, Cl or Br;
Y is $C_1$-$C_2$ alkyl, $C_1$-$C_2$ alkoxy, $OCF_2H$, $C_1$-$C_2$ alkylamino or di($C_1$-$C_2$ alkyl)amino; and

Z is CH or N.
and their agriculturally suitable salts;
provided that

a) when X is Cl or Br, then Z is CH and Y is $OCH_3$, $OCH_2CH_3$, $NHCH_3$, $N(CH_3)_2$ or $OCF_2H$; and

b) when X or Y is $OCF_2H$, then Z is CH.

The method of the invention for plantation crops more specifically involves coffee, tea, cocoa, oil palm, rubber trees, sugar cane, pineapple, plantain, banana, citrus, fruit trees, nut trees and conifers.

In the above definitions, the total number of carbon atoms in a substituent group is indicated by the $C_i$-$C_j$ prefix where i and j are numbers from 1 to 2. For example, $C_1$-$C_2$ alkyl would include methyl and ethyl, and $C_1$-$C_2$ alkoxy would include methoxy and ethoxy. $C_1$-$C_2$ alkylamino would include methylamino and ethylamino, and di($C_1$-$C_2$ alkyl)amino would include N,N-dimethylamino, N-ethyl-N-methylamino and N,N-dimethylamino.

Preferred for greater weed control efficacy, superior selectivity in maize (corn) and/or plantation crops, more favorable rate of soil degradation, and/or ease of synthesis is:

1) A method for controlling the growth of undesired vegetation which comprises applying to the locus to be protected a herbicidally effective amount of a compound of Formula I wherein
R is H or $CH_3$;
$R_1$ is $CH_3$, $CH_2CH_3$ or $(CH_2)_2CH_3$;
X is $CH_3$, $OCH_3$ or Cl;
Y is $CH_3$ or $OCH_3$; and
Z is CH or N.

2) A method as in Preferred 1 wherein
R is H;
$R_1$ is $CH_3$ or $CH_2CH_3$;
X is $OCH_3$;
Y is $CH_3$ or $OCH_3$; and
Z is CH.

3) A method as in Preferred 1 in which the compound is N-[[(4-chloro-6-methoxypyrimidin-2-yl)amino-]carbonyl]-3-(1-oxobutyl)-2-pyridinesulfonamide (Formula I, R is H, $R_1$ is $(CH_2)_2CH_3$, X is Cl, Y is $OCH_3$, Z is CH; melting range 109-112° C).

4) A method as in Preferred 2 in which the compound is N-[[(4,6-dimethoxypyrimidin2-yl)amino]carbonyl]-3-(1-oxoethyl)-2-pyridine sulfonamide (Formula I, R is H, $R_1$ is $CH_3$, X is $OCH_3$, Y is $OCH_3$, Z is CH; melting range 128-130° C).

5) A method as in Preferred 2 in which the compound is N-[[(4,6-dimethoxypyrimidin-2-yl)amino]carbonyl]-3-(1-oxopropyl)-2-pyridinesulfonamide (Formula I, R is H, $R_1$ is $CH_2CH_3$, X is $OCH_3$, Y is $OCH_3$, Z is CH; melting range 153-155° C).

6) A method as in Preferred 3 wherein the locus to be protected is a maize (corn) crop.

7) A method as in Preferred 4 wherein the locus to be protected is a plantation crop.

8) A method as in Preferred 4 wherein the locus to be protected is fallow land.

9) A method as in Preferred 5 wherein the locus to be protected is a plantation crop.

10) A method as in Preferred 9 wherein the plantation crop is citrus.

11) A method as in Preferred 9 wherein the plantation crop is sugar cane.

12) A method as in Preferred 9 wherein the plantation crop is coffee.

13) A method as in Preferred 9 wherein the plantation crop is pineapple.

14) A method as in Preferred 5 wherein the locus to be protected is fallow land.

## DETAILED DESCRIPTION OF THE INVENTION

### Synthesis

Compounds of Formula I can be prepared by one or more of the procedures shown in Equations 1, 4, 5 and 6. R, $R_1$, X, Y and Z are as previously defined.

Equation 1

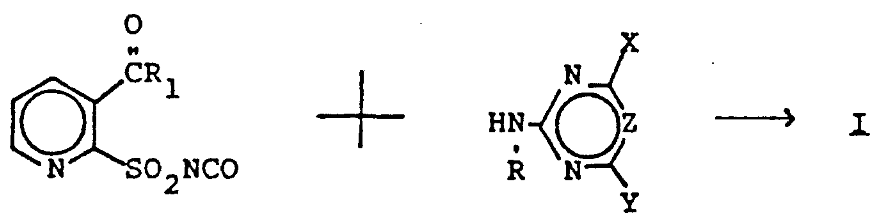

II III

The reaction of Equation 1 is best carried out in an inert aprotic organic solvent such as dichloromethane, 1,2-dichloroethane, tetrahydrofuran, or acetonitrile, at a temperature between 20° C and 85° C. The order of addition is not critical; however, it is often convenient to add the sulfonyl isocyanate or a solution of it in the reaction solvent, to a stirred suspension of the amine.

In some cases, the desired product is insoluble in the reaction solvent at ambient temperature and crystallizes from it in pure form. Products soluble in the reaction solvent are isolated by evaporation of the solvent. Compounds of Formula I then may be purified by trituration of the evaporation residue with solvents such as 1-chlorobutane or ethyl ether and filtration, by recrystallization from mixtures of solvents such as 1,2-dichloroethane, 1-chlorobutane and heptane or by chromatography on silica gel.

Sulfonyl isocyanates (II) are known in the art and are prepared from the corresponding sulfonamides (IV) by one of the following two general methods.

Equation 2

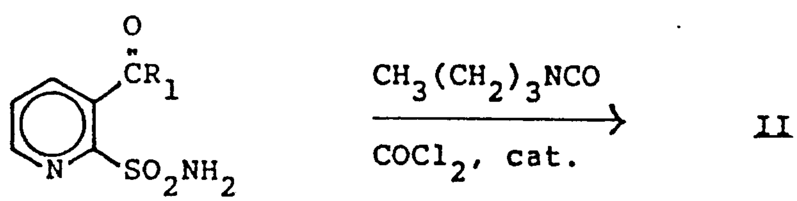


IV

The sulfonamide IV is reacted with an alkyl isocyanate (e.g., n-butyl isocyanate) in a solvent whose boiling point is above 135° C, such as xylene. The reaction can optionally be carried out in the presence of a catalytic amount of 1,4-diaza[2.2.2]-bicyclooctane (DABCO). The reaction mixture is heated to 135-140° C and held at that temperature for 5-60 minutes, after which phosgene is slowly added at such a rate that the temperature remains between 133 and 135° C. When the consumption of phosgene has ceased, the mixture is cooled and filtered to remove insoluble material. Finally, the solvent, alkyl isocyanate, and excess phosgene are evaporated, leaving the sulfonyl isocyanate (II).

If desired, the alkyl isocyanate-sulfonamide adduct can be made and isolated before reaction with the phosgene. In this case the sulfonamide (IV), alkyl isocyanate, and anhydrous base (e.g, $K_2CO_3$) in a polar, aprotic solvent (e.g. acetone, butanone, or acetonitrile) are mixed and heated under reflux for 1 to 6 hours. The reaction mixture is then diluted with water, and the pH is adjusted to about 3 with acid (e.g. HCl, $H_2SO_4$). The adduct is filtered out and dried, and then reacted with phosgene as described above. This procedure modification is especially useful when sulfonamide (IV) is high melting and has low solubility in the phosgenation solvent.

Sulfonyl isocyanates (II) can also be prepared by the following method.

Equation 3

(a) IV

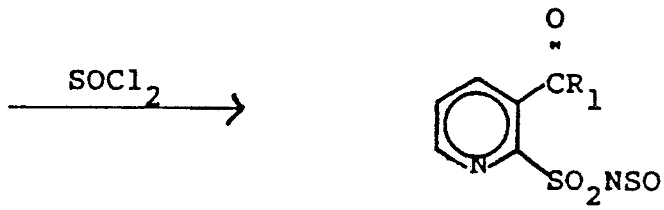


V

(b) V

$COCl_2$, pyridine cat. →

II

The sulfonamide (IV) is heated at reflux in an excess of thionyl chloride. The reaction is continued until the sulfonamide protons are no longer detectable in the proton magnetic resonance spectrum. From 16 hours to 5 days is typically sufficient for complete conversion to the thionylamide (V) (Equation 3a).

The thionyl chloride is evaporated and the residue is treated with an inert solvent (e.g. toluene containing at least one equivalent (typically 2-3 equivalents) of phosgene. A catalytic amount of pyridine (typically 0.1 equivalent) is added, and the mixture is heated to about 60-140° C, with 80-100° C preferred. Conversion to the isocyanate (II) is usually substantially complete within 15 minutes to 3 hours (Equation 3b). The mixture is then cooled and filtered, and the solvent is evaporated, leaving the sulfonyl isocyanate (II).

Many of the compounds of Formula I can be prepared by the procedure shown in Equation 4.

Equation 4

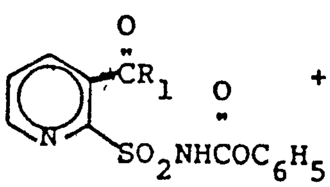

+ III → I

VI

The reaction of Equation 4 is carried out by contacting phenylcarbamates of Formula VI with aminoheterocycles of Formula III in an inert organic solvent such as dioxane or tetrahydrofuran at temperatures of about 20-100° C for a period of about one-half to twenty-four hours. The product can be isolated by evaporation of the reaction solvent and purified by methods previously described.

Phenylcarbamates of Formula VI can be prepared by the methods described, or modifications thereof known to those skilled in the art, in U.S. 4,443,243.

Alternatively, many of the compounds of Formula I can be prepared by the method described in Equation 5.

IV +

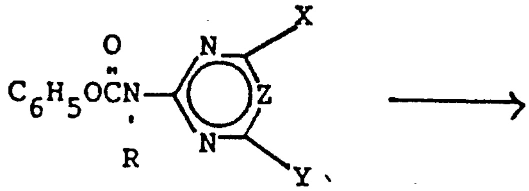

I

VII

The reaction of Equation 5 can be carried out by contacting equimolar amounts of a sulfonamide of Formula IV with a heterocyclic phenylcarbamate of Formula VII in the presence of a base such as 1,8-diazabicyclo[5.4.0]undec-7-ene (DBU), by methods analogous to those described in South African Patent Application 83/0441. The phenylcarbamates of Formula VII can be prepared by methods, or modifications thereof known to those skilled in the art, described in South African Patent Application 82/5671 and South African Patent Application 82/5045.

The sulfonamides IV of this invention may be prepared in a variety of ways known to those skilled in the art. For further details pertaining to the synthesis of pyridines see, E. Beritmaier, S. Gassenmann and E. Bayer, Tetrahedron 26, 5907 (1970); B. Blank et al., J. Med. Chem., 17, 1065 (1974); M. Mallet and G. Queguiner, Tetrahedron, 41, 3433 (1985) and J. Delarge and C.L. Lapiere, Annales Pharm. France, 36, 369 (1978).

The synthesis of heterocyclic amines such as those represented by Formula III has been reviewed in "The Chemistry of Heterocyclic Compounds," a series published by Interscience Publ., New York and London. Aminopyrimidines are described by D. J. Brown in "The Pyrimidines," Vol. XVI of the series mentioned above which is herein incorporated by reference. The 2-amino-1,3,5-triazines of Formula III, where Z is N, can be prepared according to methods described by E. M. Smolin and L. Rapaport in "s-Triazines and Derivatives," Vol. XIII.

Pyrimidines of Formula III, where A is A-1 and Y is $OCF_2H$ can be synthesized according to the methods taught in United States Patent 4,540,782.

Alternatively, the compounds of Formula I can be prepared by forming the alkanoyl carbonyl function by oxidation after forming the sulfonylurea linkage as depicted in Equation 6 and illustrated in detail in Examples 1 to 4.

Equation 6

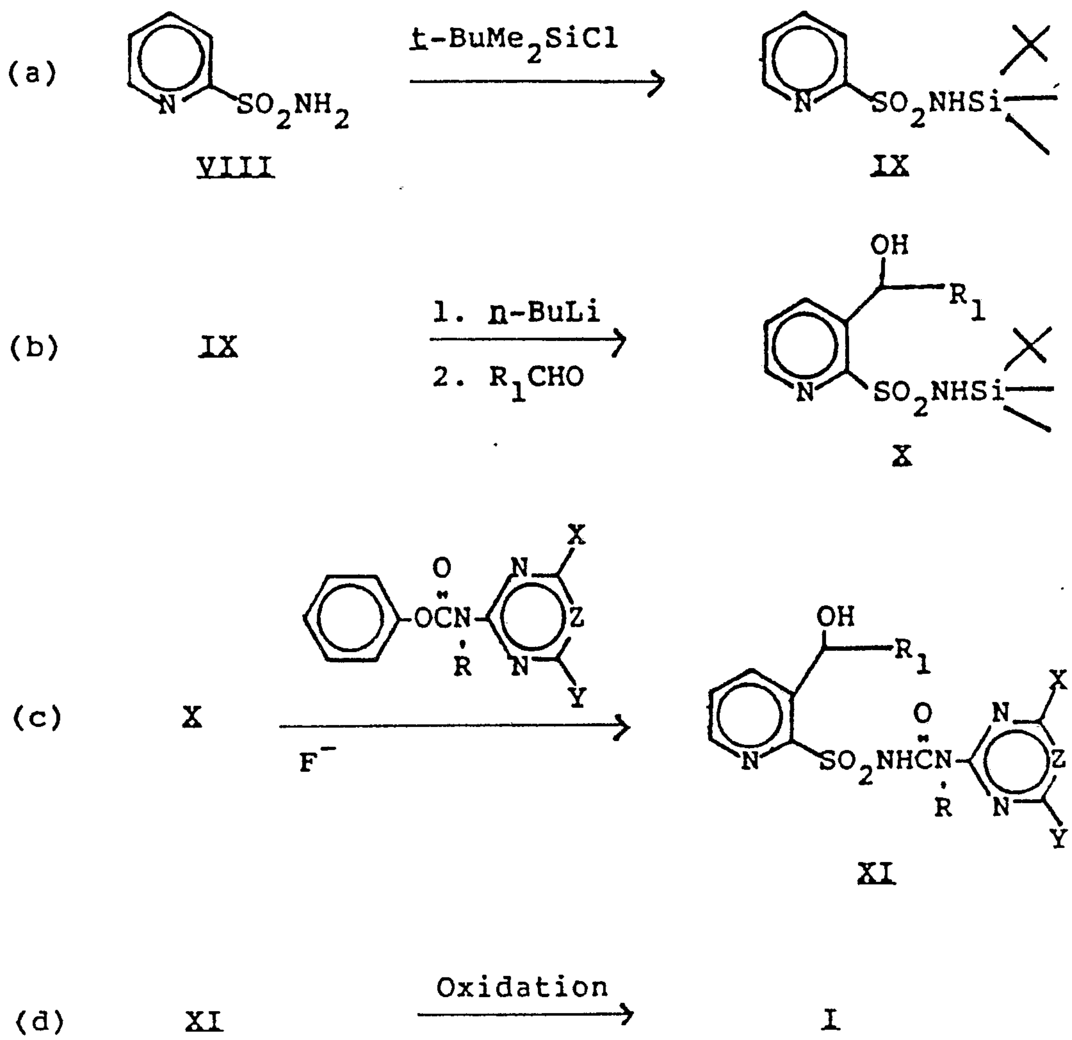


Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide, carbonate or hydroxide). Quaternary amine salts can be made by similar techniques.

A particularly useful process for preparing salts of compounds of Formula I involves reaction of a solution of the sulfonylurea in a water-immiscible halogenated hydrocarbon solvent with the appropriate alkali or alkaline earth metal hydroxide as described in copending application, U.S. Serial No. 086,867 filed on August 19, 1987.

Salts of compounds of Formula I can also be prepared by exchange of one cation for another. Cationic exchange can be effected by direct contact of an aqueous solution of a salt of a compound of Formula I (e.g. alkali or quaternary amine salt) with a solution containing the cation to be exchanged. This method is most effective when the desired salt containing the exchange cation is insoluble in water and can be separated by filtration.

Exchange may also be effected by passing an aqueous solution of a salt of a compound of Formula I (e.g., an alkali metal or quaternary amine salt) through a column packed with a cation exchange resin containing the cation to be exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula I with a suitable acid, e.g., p-toluenesulfonic acid, trichloroacetic acid or the like.

The preparation of the compounds of this invention is further illustrated by the following specific examples.

## Example 1

N-[(Dimethylethyl)dimethylsilyl]-2-pyridinesulfonamide

To a stirred solution of 2-pyridinesulfonamide (30 g, 190 mmol) and t-butyldimethylchlorosilane (43 g, 285 mmol) in methylene chloride was added triethylamine (29 g, 285 mmol) at 0 to 10°C. After stirring overnight, the reaction mixture was diluted with water, separated, dried and concentrated to afford 64 g of the title compound, m.p. 110-114°C.

## Example 2

N-[(1,1-Dimethylethyl)dimethylsilyl]-3-(1-hydroxypropyl)-2-pyridinesulfonamide

To a stirred solution of n-butylithium (6.6 g, 103 mmol) in 300 mL of tetrahydrofuran cooled to -78°C was added the pyridinesulfonamide product of Example 1 (12.5 g, 45.8 mmol). The solution was stirred for 30 minutes. Then freshly distilled proprionaldehyde (6 g, 103 mmol) was added dropwise. After warming to 50°C, the reaction mixture was quenched with brine, separated and dried. The resulting amber oil was chromatographed on silica gel (25% v/v ethyl acetatehexanes eluant) to afford 3.9 g of the title compound, m.p. 143-145°C.

## Example 3

N-[[(4,6-dimethoxypyrimidin-2-yl)amino]carbonyl]-3-(1-oxopropyl)-2-pyridinesulfonamide

Part A

To a stirred solution of the product of Example 2 (600 mg, 1.8 mmol) and the phenylcarbamate of 2-amino-4,6-dimethoxypyrimidine (500 mg, 1.8 mmol) in 5 mL of acetonitrile was added tetrabutylammonium fluoride (573 mg, 2.0 mmol). After stirring for 10 minutes, the reaction mixture was quenched with 5 mL of water, followed by 5 mL of 10% aqueous hydrochloric acid. The resulting solid was filtered and dried to afford 465 mg of the desired hydroxy sulfonylurea intermediate, m.p. 136-138°C.

Part B

To a solution of the product of Part A (450 mg, 1.1 mmol) in 50 mL of methlene chloride was added pyridinium chlorochromate (329 mg, 1.53 mmol). The reaction mixture was stirred overnight. Then 3 mL of isopropanol was added, and the reaction was stirred an additional 3 hours. The reaction mixture was then concentrated, and the resulting solids were washed with ethyl acetate. The ethyl acetate solution was subsequently concentrated to afford 185 mg of the title product, m.p. 153-155°C. Mass spectrum: 396 (M+1).

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

Weight Percent*

| | Active Ingredient | Diluent(s) | Surfactant(s) |
|---|---|---|---|
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H.M. Loux, U.S. Patent 3,235,361, February 15, 1966, Col. 6, line 16 through Col 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G.C. Klingman, "Weed Control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following Examples, all parts are by weight unless otherwise indicated.

## Example 4

Wettable Powder

| | |
|---|---|
| N-[[(4,6-dimethoxypyrimidin-2-yl)amino]carbonyl]-3-(1-oxopropyl)-2-pyridinesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

## Example 5

Wettable Powder

| | |
|---|---|
| N-[[(4,6-dimethoxypyrimidin-2-yl)amino]carbonyl]-3-(1-oxopropyl)-2-pyridinesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

## Example 6

Granule

| | |
|---|---|
| Wettable Powder of Example 5 | 5% |
| attapulgite granules (U.S.S. 20 to 40 mesh; 0.84-0.42 mm) | 95% |

A slurry of wettable powder containing 25% solids is sprayed on the surface of attapulgite granules in a double-cone blender. The granules are dried and packaged.

## Example 7

Extruded Pellet

| | |
|---|---|
| N-[[(4,6-dimethoxypyrimidin-2-yl)amino]carbonyl]-3-(1-oxopropyl)-2-pyridinesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 8

Low Strength Granule

| | |
|---|---|
| N-[[(4,6-dimethoxypyrimidin-2-yl)amino]carbonyl]-3-(1-oxopropyl)-2-pyridinesulfonamide | 0.1% |
| attapulgite granules (U.S.S. 20 to 40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 9

Granule

| | |
|---|---|
| N-[[(4,6-dimethoxypyrimidin-2-yl)amino]carbonyl]-3-(1-oxopropyl)-2-pyridinesulfonamide | 80% |
| wetting agent | 1% |
| crude ligninsulfonate salt (containing 5 to 20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14 to 100 mesh (1410 to 149 microns), and packaged for use.

Example 10

Low Strength Granule

| | |
|---|---|
| N-[[(4,6-dimethoxypyrimidin-2-yl)amino]carbonyl]-3-(1-oxopropyl)-2-pyridinesulfonamide | 1% |
| N,N-dimethylformamide | 9% |
| attapulgite granules (U.S.S. 20 to 40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 11

Aqueous Suspension

| | |
|---|---|
| N-[[(4,6-dimethoxypyrimidin-2-yl)amino]carbonyl]-3-(1-oxopropyl)-2-pyridinesulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 12

Solution

| | |
|---|---|
| N-[[(4,6-dimethoxypyrimidin-2-yl)amino]carbonyl]-3-(1-oxopropyl)-2-pyridinesulfonamide ammonium salt | 5% |
| water | 95% |

The salt is added directly to the water with stirring to produce the solution, which may then be packaged for use.

Example 13

High Strength Concentrate

| | |
|---|---|
| N-[[(4,6-dimethoxypyrimidin-2-yl)amino]carbonyl]-3-(1-oxopropyl)-2-pyridinesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 14

Wettable Powder

| | |
|---|---|
| N-[[(4,6-dimethoxypyrimidin-2-yl)amino]carbonyl]-3-(1-oxopropyl)-2-pyridinesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 15

Wettable Powder

| | |
|---|---|
| N-[[(4,6-dimethoxypyrimidin-2-yl)amino]carbonyl]-3-(1-oxopropyl)-2-pyridinesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 16

Oil Suspension

| | |
|---|---|
| N-[[(4,6-dimethoxypyrimidin-2-yl)amino]carbonyl]-3-(1-oxopropyl)-2-pyridinesulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 17

Dust

| | |
|---|---|
| N-[[(4,6-dimethoxypyrimidin-2-yl)amino]carbonyl]-3-(1-oxopropyl)-2-pyridinesulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example 18

Oil Suspension

| | |
|---|---|
| N-[[(4,6-dimethoxypyrimidin-2-yl)amino]carbonyl]-3-(1-oxopropyl)-2-pyridinesulfonamide | 25% |
| polyoxyethylene sorbitol hexaoleate | 5% |
| highly aliphatic hydrocarbon oil | 70% |

The ingredients are ground together in a sand mill until the solid particles have been reduced to under

about 5 microns. The resulting thick suspension may be applied directly, but preferably after being extended with oils or emulsified in water.

## Example 19

Wettable Powder

| | |
|---|---|
| N-[[(4,6-dimethoxypyrimidin-2-yl)amino]carbonyl]-3-(1-oxopropyl)-2-pyridinesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Utility

The compounds of this invention are active herbicides useful for selective and nonselective broadleaf and grass control in crop and non-crop situations wherein the crop applications include maize (corn), soybeans, plantation crops such as coffee, tea, cocoa, oil palm, rubber tree, sugarcane, pineapple, plantain, banana, citrus, fruit and nut trees and conifers, and the non-crop application includes fallow situations where control of all vegetation is desired.

The compounds may be applied either preemergence or postemergence using directed sprays, broadcast applications, or handling techniques necessary to provide the desired weed control and crop selectivity. These chemicals can be used to control vegetation in fallow fields and sensitive crops planted after a reasonable interval. That interval will depend on soil, climate and season but can be developed for any situation by one skilled in the art. With appropriate rates, these materials may be used to control all vegetation in industrial areas, along right-of-ways, roadsides, etc.

In general, the compounds of this invention are used at rates of 0.25 to 10,000 g/ha. The preferred rates are 5 to 1,000 g/ha. The exact rate that should be used will depend on the weeds to be controlled, the soil type, the method of application, the climate, the presence or absence of a crop and the size and vigor of any weeds present. One with ordinary skill in the art can select the appropriate rate.

The compounds of this invention may be used in combination with other herbicides. A partial list of herbicides that may be combined with these chemicals is given below.

| Common Name | Chemical Name |
|---|---|
| acetochlor | 2-chloro-N-(ethoxymethyl)-N-(2-ethyl-6-methylphenyl)acetamide |
| acifluorfen | 5-[2-chloro-4-(trifluoromethyl)phenoxy]-2-nitrobenzoic acid |
| acrolein | 2-propenal |
| alachlor | 2-chloro-N-(2,6-diethylphenyl)-N-(methoxymethyl)acetamide |
| ametryn | N-ethyl-N'-(1-methylethyl)-6-(methylthio)-1,3,5-triazine-2,4-diamine |
| amitrole | 1H-1,2,4-triazol-3-amine |
| AMS | ammonium sulfamate |
| asulam | methyl [(4-aminophenyl)sulfonyl]-carbamate |
| atrazine | 6-chloro-N-ethyl-N'-(1-methylethyl)-1,3,5-triazine-2,4-diamine |
| barban | 4-chloro-2-butynyl 3-chlorocarbamate |
| benefin | N-butyl-N-ethyl-2,6-dinitro-4-(trifluoromethyl)benzenamine |
| bensulfuron | 2-[[[[[4,6-dimethoxy-2-pyrimidinyl) amino]-methylcarbonyl]amino]sulfonyl] methyl]benzoic acid, methyl ester |
| bensulide | O,O-bis(1-methylethyl) S-[2-[(phenylsulfonyl)amino]ethyl] phosphorodithioate |
| bentazon | 3-(1-methylethyl)-(1H)-2,1,3-benzothiadiazin-4(3H)-one, 2,2-dioxide |
| benzofluor | N-[4-(ethylthio)-2-(trifluoromethyl)-phenyl]methanesulfonamide |
| benzoylprop | N-benzoyl-N-(3,4-dichlorophenyl)-DL-alanine |

| Common Name | Chemical Name |
|---|---|
| bifenox | methyl 5-(2,4-dichlorophenoxy)-2-nitrobenzoate |
| bromacil | 5-bromo-6-methyl-3-(1-methylpropyl)-2,4(1H,3H)pyrimidinedione |
| bromoxynil | 3,5-dibromo-4-hydroxybenzonitrile |
| butachlor | N-(butoxymethyl)-2-chloro-N-(2,6-diethyl-phenyl)acetamide |
| buthidazole | 3-[5-(1,1-dimethylethyl)-1,3,4-thiadiazol-2-yl]-4-hydroxy-1-methyl-2-imidazolidinone |
| butralin | 4-(1,1-dimethylethyl)-N-(1-methylpropyl)-2,6-dinitrobenzenamine |
| butylate | S-ethyl bis(2-methylpropyl)carbamothioate |
| cacodylic | dimethyl arsinic oxide acid |
| CDAA | 2-chloro-N,N-di-2-propenylacetamide |
| CDEC | 2-chloroallyl diethyldithiocarbamate |
| chloramben | 3-amino-2,5-dichlorobenzoic acid |
| chlorbromuron | 3-(4-bromo-3-chlorophenyl)-1-methoxy-1-methylurea |
| chlorimuron | 2-[[[[(4-chloro-6-methoxy-2-pyrimidinyl)-ethylamino]carbonyl]amino]sulfonyl]benzoic acid, ethyl ester |
| chloroxuron | N'-[4-(4-chlorophenoxy)phenyl]-N,N-dimethylurea |
| chlorpropham | 1-methylethyl 3-chlorophenylcarbamate |
| chlorsulfuron | 2-chloro-N-[[(4-methoxy-6-methyl-1,3,5-triazin-2-yl)amino]carbonyl]benzenesulfonamide |
| chlortoluron | N'-(3-chloro-4-methylphenyl)-N,N-dimethylurea |

| Common Name | Chemical Name |
|---|---|
| cinmethylin | exo-1-methyl-4-(1-methylethyl)-2-[(2-methylphenyl)methoxy]-7-oxabicyclo-[2.2.1]heptane |
| clethodim | (E,E)-(±)-2-[1-[[(3-chloro-2-propenyl)-oxy]imino]propyl]-5-[2-(ethylthio)-propyl]-3-hydroxy-2-cyclohexen-1-one |
| clomazone | 2-[(2-chlorophenyl)methyl]-4,4-dimethyl-3-isoxazolidinone |
| cloproxydim | (E,E)-2-[1-[[(3-chloro-2-propenyl)oxy)-imino]butyl]-5-[2-(ethylthio)propyl]-3-hydroxy-2-cyclohexen-1-one |
| clopyralid | 3,6-dichloro-2-pyridinecarboxylic acid |
| CMA | calcium salt of MAA |
| cyanazine | 2-[[4-chloro-6-(ethylamino)-1,3,5-tri-azin-2-yl]amino]-2-methylpropanenitrile |
| cycloate | S-ethyl cyclohexylethylcarbamothioate |
| cycluron | 3-cyclooctyl-1,1-dimethylurea |
| cyperquat | 1-methyl-4-phenylpyridinium |
| cyprazine | 2-chloro-4-(cyclopropylamino)-6-(iso-propylamino)-s-triazine |
| cyprazole | N-[5-(2-chloro-1,1-dimethylethyl)-1,3,4-thiadiazol-2-yl]cyclopropanecarboxamide |
| cypromid | 3',4'-dichlorocyclopropanecarboxanilide |
| dalapon | 2,2-dichloropropanoic acid |
| dazomet | tetrahydro-3,5-dimethyl-2H-1,3,5-thia-diazine-2-thione |
| DCPA | dimethyl 2,3,5,6-tetrachloro-1,4-benzenedicarboxylate |
| desmediphan | ethyl [3-[[(phenylamino)carbonyl]oxy]-phenyl]carbamate |

| Common Name | Chemical Name |
|---|---|
| desmetryn | 2-(isopropylamino)-4-(methylamino)-6-(methylthio)-s-triazine |
| diallate | S-(2,3-dichloro-2-propenyl)bis(1-methylethyl)carbamothioate |
| dicamba | 3,6-dichloro-2-methoxybenzoic acid |
| dichlobenil | 2,6-dichlorobenzonitrile |
| dichlorprop | (±)-2-(2,4-dichlorophenoxy)propanoic acid |
| dichlofop | (±)-2-[4-(2,4-dichlorophenoxy)phenoxy]-propanoic acid |
| diethatyl | N-(chloroacetyl)-N-(2,6-diethylphenyl)-glycine |
| difenzoquat | 1,2-dimethyl-3,5-diphenyl-1H-pyrazolium |
| dinitramine | $N^3$,$N^3$-diethyl-2,4-dinitro-6-(trifluoromethyl)-1,3-benzenediamine |
| dinoseb | 2-(1-methylpropyl)-4,6-dinitrophenol |
| diphenamid | N,N-dimethyl-α-phenylbenzeneacetamide |
| dipropetryn | 6-(ethylthio)-N,N'-bis(1-methylethyl)-1,3,5-triazine-2,4-diamine |
| diquat | 6,7-dihydrodipyrido[1,2-a:2',1'-c]-pyrazinedium ion |
| diuron | N'-(3,4-dichlorophenyl)-N,N-dimethylurea |
| DNOC | 2-methyl-4,6-dinitrophenol |
| DPX-M6316 | 3-[[[[(4-methoxy-6-methyl-1,3,5-triazin-2-yl)amino]carbonyl]amino]sulfonyl]-2-thiophenecarboxylic acid, methyl ester |
| DSMA | disodium salt of MAA |
| endothall | 7-oxabicyclo[2.2.1]heptane-2,3-dicarboxylic acid |
| EPTC | S-ethyl dipropylcarbamothioate |

| Common Name | Chemical Name |
|---|---|
| ethalfluralin | N-ethyl-N-(2-methyl-2-propenyl)-2,6-dinitro-4-(trifluoromethyl)benzenamine |
| ethofumesate | (±)-2-ethoxy-2,3-dihydro-3,3-dimethyl-5-benzofuranyl methanesulfonate |
| Express® | 2-[[[[N-(4-methoxy-6-methyl-1,3,5-triazine-2-yl)-N-methylamino]carbonyl]amino]- sulfonyl]benzoic acid, methyl ester |
| fenac | 2,3,6-trichlorobenzeneacetic acid |
| fenoxaprop | (±)-2-[4-[(6-chloro-2-benzoxazolyl)oxy]-phenoxy]propanoic acid |
| fenuron | N,N-dimethyl-N'-phenylurea |
| fenuron TCA | Salt of fenuron and TCA |
| flamprop | N-benzoyl-N-(3-chloro-4-fluorophenyl)-DL-alanine |
| fluazifop | (±)-2-[4-[[5-(trifluoromethyl)-2-pyridinyl]oxy]phenoxy]propanoic acid |
| fluazifop-P | (R)-2-[4-[[5-(trifluoromethyl)-2-pyridinyl]oxy]phenoxy]propanoic acid |
| fluchloralin | N-(2-chloroethyl)-2,6-dinitro-N-propyl-4-(trifluoromethyl)benzenamine |
| fluometuron | N,N-dimethyl-N'-[3-(trifluoromethyl)-phenyl]urea |
| fluorochloridone | 3-chloro-4-(chloromethyl)-1-[3-(trifluoromethyl)phenyl]-2-pyrrolidinone |
| fluorodifen | p-nitrophenyl α,α,α-trifluoro-2-nitro-p-tolyl ether |
| fluoroglycofen | carboxymethyl 5-[2-chloro-4-(trifluoromethyl)phenoxy]-2-nitrobenzoate |
| fluridone | 1-methyl-3-phenyl-5-[3-(trifluoromethyl)phenyl]-4(1H)-pyridinone |
| fomesafen | 5-[2-chloro-4-(trifluoromethyl)phenoxy]-N-(methylsulfonyl)-2-nitrobenzamide |

| Common Name | Chemical Name |
|---|---|
| fosamine | ethyl hydrogen (aminocarbonyl)-phosphate |
| glyphosate | N-(phosphonomethyl)glycine |
| haloxyfop | 2-[4-[[3-chloro-5-(trifluoromethyl)-2-pyridinyl]oxy]phenoxy]propanoic acid |
| hexaflurate | potassium hexafluoroarsenate |
| hexazinone | 3-cyclohexyl-6-(dimethylamino)-1-methyl-1,3,5-triazine-2,4(1H,3H)-dione |
| imazamethabenz | 6-(4-isopropyl-4-methyl-5-oxo-2-imidazolin-2-yl)-m-toluic acid, methyl ester and 6-(4-isopropyl-4-methyl-5-oxo-2-imidazolin-2-yl)-p-toluic acid, methyl ester |
| imazapyr | (±)-2-[4,5-dihydro-4-methyl-4-(1-methylethyl)-5-oxo-1H-imidazol-2-yl]-3-pyridinecarboxylic acid |
| imazaquin | 2-[4,5-dihydro-4-methyl-4-(1-methylethyl)-5-oxo-1H-imidazol-2-yl]-3-quinolinecarboxylic acid |
| imazethapyr | (±)-2-[4,5-dihydro-4-methyl-4-(1-methylethyl)-5-oxo-1H-imidazol-2-yl]-5-ethyl-3-pyridinecarboxylic acid |
| ioxynil | 4-hydroxy-3,5-diiodobenzonitrile |
| isopropalin | 4-(1-methylethyl)-2,6-dinitro-N,N-dipropylbenzenamine |
| isoproturon | N-(4-isopropylphenyl)-N',N'-dimethylurea |
| isouron | N'-[5-(1,1-dimethylethyl)-3-isoxazolyl]-N,N-dimethylurea |
| isoxaben | N-[3-(1-ethyl-1-methylpropyl)-5-isoxazolyl]-2,6-dimethoxybenzamide |
| karbutilate | 3-[[(dimethylamino)carbonyl]amino]-phenyl-(1,1-dimethylethyl)carbamate |

| Common Name | Chemical Name |
|---|---|
| lactofen | (±)-2-ethoxy-1-methyl-2-oxoethyl 5-[2-chloro-4-(trifluoromethyl)phenoxy]-2-nitrobenzoate |
| lenacil | 3-cyclohexyl-6,7-dihydro-1H-cyclopentapyrimidine-2,4(3H,5H)-dione |
| linuron | N'-(3,4-dichlorophenyl)-N-methoxy-N-methylurea |
| MAA | methylarsonic acid |
| MAMA | monoammonium salt of MAA |
| MCPA | (4-chloro-2-methylphenoxy)acetic acid |
| MCPB | 4-(4-chloro-2-methylphenoxy)butanoic acid |
| mecoprop | (±)-2-(4-chloro-2-methylphenoxy)-propanoic acid |
| mefluidide | N-[2,4-dimethyl-5-[[(trifluoromethyl)-sulfonyl]amino]phenyl]acetamide |
| methal-propalin | N-(2-methyl-2-propenyl)-2,6-dinitro-N-propyl-4-(trifluoromethyl)benzenamide |
| methabenz-thiazuron | 1,3-dimethyl-3-(2-benzothiazolyl)urea |
| metham | methylcarbamodithioic acid |
| methazole | 2-(3,4-dichlorophenyl)-4-methyl-1,2,4-oxadiazolidine-3,5-dione |
| methoxuron | N'-(3-chloro-4-methoxyphenyl)-N,N-dimethylurea |
| metolachlor | 2-chloro-N-(2-ethyl-6-methylphenyl)-N-(2-methoxy-1-methylethyl)acetamide |
| metribuzin | 4-amino-6-(1,1-dimethylethyl)-3-(methylthio)-1,2,4-triazin-5(4H)-one |
| metsulfuron methyl | 2-[[[[(4-methoxy-6-methyl-1,3,5-triazin-2-yl)amino]carbonyl]-amino]sulfonyl]benzoic acid, methyl ester |

| Common Name | Chemical Name |
|---|---|
| MH | 1,2-dihydro-3,6-pyridazinedione |
| molinate | S-ethyl hexahydro-1H-azepine-1-carbothioate |
| monolinuron | 3-(p-chlorophenyl)-1-methoxy-1-methylurea |
| monuron | N'-(4-chlorophenyl)-N,N-dimethylurea |
| monuron TCA | Salt of monuron and TCA |
| MSMA | monosodium salt of MAA |
| napropamide | N,N-diethyl-2-(1-naphthalenyloxy)-propanamide |
| naptalam | 2-[(1-naphthalenylamino)carbonyl]-benzoic acid |
| neburon | 1-butyl-3-(3,4-dichlorophenyl)-1-methylurea |
| nitralin | 4-(methylsulfonyl)-2,6-dinitro-N,N-dipropylaniline |
| nitrofen | 2,4-dichloro-1-(4-nitrophenoxy)benzene |
| nitrofluorfen | 2-chloro-1-(4-nitrophenoxy)-4-(trifluoromethyl)benzene |
| norea | N,N-dimethyl-N'-(octahydro-4,7-methano-1H-inden-5-yl)urea 3aα,-4α,5α,7α,7aα-isomer |
| norflurazon | 4-chloro-5-(methylamino)-2-[3-(trifluoromethyl)phenyl]-3(2H)-pyridazinone |
| oryzalin | 4-(dipropylamino)-3,5-dinitrobenzenesulfonamide |
| oxadiazon | 3-[2,4-dichloro-5-(1-methylethoxy)-phenyl]-5-(1,1-dimethylethyl)-1,3,4-oxadiazol-2(3H)-one |
| oxyfluorfen | 2-chloro-1-(3-ethoxy-4-nitrophenoxy)-4-(trifluoromethyl)benzene |

| Common Name | Chemical Name |
|---|---|
| paraquat | 1,1'-dimethyl-4,4'-dipyridinium ion |
| pebulate | S-propyl butylethylcarbamothioate |
| pendimethalin | N-(1-ethylpropyl)-3,4-dimethyl-2,6-dinitrobenzenamine |
| perfluidone | 1,1,1-trifluoro-N-[2-methyl-4-(phenylsulfonyl)phenyl]methanesulfonamide |
| phenmedipham | 3-[(methoxycarbonyl)amino]phenyl (3-methylphenyl)carbamate |
| picloram | 4-amino-3,5,6-trichloro-2-pyridinecarboxylic acid |
| PPG-1013 | 5-[2-chloro-4-(trifluoromethyl)-phenoxy]-2-nitroacetophenone oxime-O-acetic acid, methyl ester |
| procyazine | 2-[[4-chloro-6-(cyclopropylamino)-1,3,5-triazine-2-yl]amino]-2-methylpropanenitrile |
| profluralin | N-(cyclopropylmethyl)-2,6-dinitro-N-propyl-4-(trifluoromethyl)benzenamine |
| prometon | 6-methoxy-N,N'-bis(1-methylethyl)-1,3,5-triazine-2,4-diamine |
| prometryn | N,N'-bis(1-methylethyl)-6-(methylthio)-1,3,5-triazine-2,4-diamine |
| pronamide | 3,5-dichloro-N-(1,1-dimethyl-2-propynyl)benzamide |
| propachlor | 2-chloro-N-(1-methylethyl)-N-phenylacetamide |
| propanil | N-(3,4-dichlorophenyl)propanamide |
| propazine | 6-chloro-N,N'-bis(1-methylethyl)-1,3,5-triazine-2,4-diamine |
| propham | 1-methylethyl phenylcarbamate |

| Common Name | Chemical Name |
|---|---|
| prosulfalin | N-[[4-(dipropylamino)-3,5-dinitrophenyl]sulfonyl]-S,S-dimethylsulfilimine |
| prynachlor | 2-chloro-N-(1-methyl-2-propynyl)acetanilide |
| pyrazon | 5-amino-4-chloro-2-phenyl-3(2H)-pyridazinone |
| quizalofop ethyl | (±)-2-[4-[(6-chloro-2-quinoxalinyl)-oxy]phenoxy]propanoic acid, ethyl ester |
| secbumeton | N-ethyl-6-methoxy-N'-(1-methylpropyl)-1,3,5-triazine-2,4-diamine |
| sethoxydim | 2-[1-(ethoxyimino)butyl]-5-[2-(ethylthio)propyl]-3-hydroxy-2-cyclohexen-1-one |
| siduron | N-(2-methylcyclohexyl)-N'-phenylurea |
| simazine | 6-chloro-N,N'-diethyl-1,3,5-triazine-2,4-diamine |
| sulfometuron methyl | 2-[[[[(4,6-dimethyl-2-pyrimidinyl)-amino]carbonyl]amino]sulfonyl]-benzoic acid, methyl ester |
| TCA | trichloroacetic acid |
| tebuthiuron | N-[5-(1,1-dimethylethyl)-1,3,4-thiadiazol-2-yl]-N,N'-dimethylurea |
| terbacil | 5-chloro-3-(1,1-dimethylethyl)-6-methyl-2,4(1H,3H)-pyrimidinedione |
| terbuchlor | N-(butoxymethyl)-2-chloro-N-[2-(1,1-dimethylethyl)-6-methylphenyl]-acetamide |
| terbuthylazine | 2-(*tert*-butylamino)-4-chloro-6-(ethylamino)-*s*-triazine |
| terbutol | 2,6-di-*tert*-butyl-*p*-tolyl methylcarbamate |

| Common Name | Chemical Name |
|---|---|
| terbutryn | N-(1,1-dimethylethyl)-N'-ethyl-6-(methylthio)-1,3,5-triazine-2,4-diamine |
| thiobencarb | S-[(4-chlorophenyl)methyl] diethylcarbamothioate |
| triallate | S-(2,3,3-trichloro-2-propenyl) bis(1-methylethyl)carbamothioate |
| triclopyr | [(3,5,6-trichloro-2-pyridinyl)-oxy]acetic acid |
| tridiphane | 2-(3,5-dichlorophenyl)-2-(2,2,2-trichloroethyl)oxirane |
| trifluralin | 2,6-dinitro-N,N-dipropyl-4-(trifluoromethyl)benzenamine |
| trimeturon | 1-(p-chlorophenyl)-2,3,3-trimethylpseudourea |
| 2,4-D | (2,4-dichlorophenoxy)acetic acid |
| 2,4-DB | 4-(2,4-dichlorophenoxy)butanoic acid |
| vernolate | S-propyl dipropylcarbamothioate |
| xylachlor | 2-chloro-N-(2,3-dimethylphenyl)-N-(1-methylethyl)acetamide |

The herbicidal properties of the subject compounds were discovered in a number of greenhouse tests. The test procedures and results follow.

COMPOUNDS

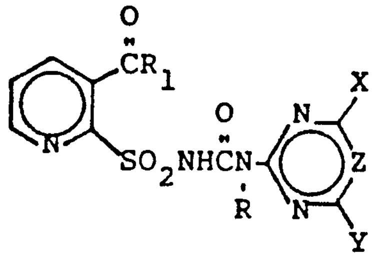

| Compound | R | $R_1$ | X | Y | Z | m.p.(°C) |
|---|---|---|---|---|---|---|
| 1 | H | $CH_2CH_3$ | $CH_3$ | $CH_3$ | CH | 154-155 |
| 2 | H | $CH_2CH_3$ | $CH_3$ | $OCH_3$ | CH | 100-103 |
| 3 | H | $CH_2CH_3$ | $OCH_3$ | $OCH_3$ | CH | 153-155 |
| 4 | H | $CH_2CH_3$ | $CH_3$ | $OCH_3$ | N | 144-146 |
| 5 | H | $CH_2CH_3$ | $OCH_3$ | $OCH_3$ | N | 140-142 |
| 6 | H | $CH_2CH_3$ | Cl | $OCH_3$ | CH | 148-150 |
| 7 | H | $CH_3$ | $OCH_3$ | $OCH_3$ | CH | 128-130 |
| 8 | H | $CH_3$ | Cl | $OCH_3$ | CH | 141-142 |
| 9 | H | $(CH_2)_2CH_3$ | $CH_3$ | $CH_3$ | CH | 136-137 (d) |
| 10 | H | $(CH_2)_2CH_3$ | $OCH_3$ | $OCH_3$ | CH | 136-137 (d) |
| 11 | H | $(CH_2)_2CH_3$ | $OCH_3$ | $OCH_3$ | N | 123-124 (d) |
| 12 | H | $(CH_2)_2CH_3$ | Cl | $OCH_3$ | CH | 109-112 |
| 13 | H | $(CH_2)_2CH_3$ | $CH_3$ | $OCH_3$ | CH | 111-115 (d) |

Test A

Seeds of crabgrass (Digitaria spp.), barnyardgrass (Echinochloa crus-galli), giant foxtail (Setaria faberi), wild oats (Avena fatua), cheatgrass (Bromus secalinus), velvetleaf (Abutilon theophrasti), morningglory (Ipomoea spp.), cocklebur (Xanthium pensylvanicum), sorghum, corn, soybean, sugarbeet, cotton, rice, wheat, barley and purple nutsedge (Cyperus rotundus) tubers were planted and treated preemergence with the test chemicals dissolved in a non-phytotoxic solvent. At the same time, these crop and weed species were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

C = chlorosis/necrosis
B = burn
D = defoliation
E = emergence inhibition
G = growth retardation
H = formative effect
U = unusual pigmentation
X = axillary stimulation
S = albinism
6Y = abscised buds or flowers

TABLE A

| | CMPD 1 | | CMPD 2 | |
|---|---|---|---|---|
| RATE=KG/HA | 0.05 | 0.01 | 0.05 | 0.01 |
| POSTEMERGENCE | | | | |
| COTTON | 5C,9G | 3C,9G | 5C,9G | 9C |
| MORNINGGLORY | 10C | 3C,8G | 9C | 3C,8H |
| COCKLEBUR | 7G | 0 | 5C,9G | 3C,7H |
| NUTSEDGE | 10C | 4C,8G | 7G | 3G |
| CRABGRASS | 10C | 3C,5G | 3C,7G | 2C,4G |
| BARNYARDGRASS | 9C | 4C,9H | 9C | 3C,8G |
| WILD OATS | 5C,9G | 9G | 3C,8G | 6G |
| WHEAT | 5C,9G | 4C,9G | 5C,9G | 8G |
| CORN | 1C,3G | 0 | 4C,9H | 3C,6H |
| SOYBEANS | 5C,9G | 3C,6G | 5C,9G | 4C,9G |
| RICE | 4C,9G | 5C,9G | 5C,9G | 5C,9G |
| SORGHUM | 4C,9G | 3C,7G | 3C,8G | 3C,7H |
| CHEATGRASS | 5C,9G | 3C,7G | 3C,7G | 6G |
| SUGARBEETS | 10C | 5C,9G | 5C,9G | 4C,9G |
| VELVETLEAF | 5C,9G | 3C,8G | 5C,9G | 4C,9H |
| GIANT FOXTAIL | 3C,7G | 2G | 3C,7G | 3C,6G |
| BARLEY | 8G | 2C,7G | 6G | 3G |

TABLE A

| | CMPD 1 | | CMPD 2 | |
|---|---|---|---|---|
| RATE=KG/HA | 0.05 | 0.01 | 0.05 | 0.01 |
| PREEMERGENCE | | | | |
| COTTON | 4G | 2G | 0 | 0 |
| MORNINGGLORY | 9G | 3C,3G | 2C | 0 |
| COCKLEBUR | 8H | 3G | 1C | 0 |
| NUTSEDGE | 4G | 0 | 0 | 0 |
| CRABGRASS | 3C,7G | 0 | 4G | 0 |
| BARNYARDGRASS | 2G | 0 | 2C,2G | 0 |
| WILD OATS | 3C,5G | 3G | 0 | 0 |
| WHEAT | 9G | 7G | 5G | 2G |
| CORN | 3G | 0 | 3C,4G | 0 |
| SOYBEANS | 3C,9H | 3C,3H | 3C,5H | 2C,2H |
| RICE | 10E | 5G | 3C,3G | 1C |
| SORGHUM | 3C,9H | 3C,7G | 3C,5G | 2C |
| CHEATGRASS | 7G | 2G | 4G | 0 |
| SUGARBEETS | 8G | 5G | 5H | 3G |
| VELVETLEAF | 3C,7G | 2H | 1C,1H | 0 |
| GIANT FOXTAIL | 2G | 0 | 0 | 0 |
| BARLEY | 9G | 2C,4G | 3G | 0 |

TABLE A

| | CMPD 3 | | CMPD 4 | |
|---|---|---|---|---|
| RATE=KG/HA | 0.05 | 0.01 | 0.05 | 0.01 |
| POSTEMERGENCE | | | | |
| COTTON | 9C | 5C,9G | 3C,7G | 3C,5G |
| MORNINGGLORY | 10C | 10C | 9C | 3C,7H |
| COCKLEBUR | 10C | 9C | 8H | 3H,5G |
| NUTSEDGE | 10C | 10C | 5C,5G | 5G |
| CRABGRASS | 10C | 5C,9G | 4C,9G | 3C,5G |
| BARNYARDGRASS | 10C | 10C | 9C | 4C,9H |
| WILD OATS | 4C,9G | 2C,9G | 4C,9G | 4C,9G |
| WHEAT | 10C | 9C | 9C | 5C,9G |
| CORN | 9C | 3C,9H | 3C,9G | 3C,7H |
| SOYBEANS | 9C | 9C | 4C,9G | 3H,5G |
| RICE | 9C | 5C,9G | 5C,9G | 5C,9G |
| SORGHUM | 9C | 9C | 5C,9G | 3C,9G |
| CHEATGRASS | 10C | 5C,9G | 5C,9G | 8G |
| SUGARBEETS | 10C | 10C | 10C | 9C |
| VELVETLEAF | 10C | 10C | 7H | 2H |
| GIANT FOXTAIL | 10C | 9C | 5C,9G | 4C,9G |
| BARLEY | 9C | 3C,9G | 9C | 3C,8G |

TABLE A

| | CMPD 3 | | CMPD 4 | |
|---|---|---|---|---|
| RATE=KG/HA | 0.05 | 0.01 | 0.05 | 0.01 |
| PREEMERGENCE | | | | |
| COTTON | 9G | 8G | 2G | 0 |
| MORNINGGLORY | 5C,9G | 3C,9H | 3C,4G | 0 |
| COCKLEBUR | 4C,8H | 3C,7G | 0 | 0 |
| NUTSEDGE | 10E | 0 | 0 | - |
| CRABGRASS | 4C,9G | 3C,8G | 3C,7G | 0 |
| BARNYARDGRASS | 9H | 3C,9H | 7G | 5H |
| WILD OATS | 2C,8G | 3C,7G | 4G | 0 |
| WHEAT | 9G | 8G | 9H | 7G |
| CORN | 3C,9H | 3C,6G | 3C,9G | 3C,7G |
| SOYBEANS | 9H | 3C,8H | 3C,5G | 0 |
| RICE | 10E | 8G | 10H | 9H |
| SORGHUM | 5C,9H | 3C,9G | 10H | 3C,9G |
| CHEATGRASS | 9H | 8G | 3G | 3G |
| SUGARBEETS | 4C,9G | 9G | 7H | 3H |
| VELVETLEAF | 3C,8G | 2C,7G | 0 | 0 |
| GIANT FOXTAIL | 3C,9H | 3C,9H | 2G | 0 |
| BARLEY | 9G | 2C,9G | 2C,9G | 3C,7G |

TABLE A

| | CMPD 5 | | CMPD 6 | |
|---|---|---|---|---|
| RATE=KG/HA | 0.05 | 0.01 | 0.05 | 0.01 |
| POSTEMERGENCE | | | | |
| COTTON | 4C,9H | 3C,6G | 4C,9G | 5C,9G |
| MORNINGGLORY | 5C,9G | 3C,8H | 9C | 10C |
| COCKLEBUR | 8G | 2H | 10C | 10C |
| NUTSEDGE | 0 | 0 | 10C | 10C |
| CRABGRASS | 9C | 3C,7G | 10C | 3C,7G |
| BARNYARDGRASS | 10C | 4C,9H | 9C | 10C |
| WILD OATS | 5C,9G | 4C,9G | 2C,9G | 4G |
| WHEAT | 9C | 9C | 3C,9G | 5G |
| CORN | 9C | 4C,9G | 2C,3H | 0 |
| SOYBEANS | 5C,9G | 2C,5H | 4C,9G | 3C,8H |
| RICE | 9C | 5C,9G | 5C,9G | 4C,9G |
| SORGHUM | 9C | 3C,8G | 10C | 10C |
| CHEATGRASS | 9C | 5C,9G | 9C | 8G |
| SUGARBEETS | 9C | 4C,9G | 10C | 10C |
| VELVETLEAF | 3C,7H | 4G | 4C,9H | 4C,8H |
| GIANT FOXTAIL | 9C | 5C,9G | 9C | 3C,8G |
| BARLEY | 5C,9G | 3C,9G | 9G | 8G |

TABLE A

| | CMPD 5 | | CMPD 6 | |
|---|---|---|---|---|
| RATE=KG/HA | 0.05 | 0.01 | 0.05 | 0.01 |
| PREEMERGENCE | | | | |
| COTTON | 2G | 0 | 3C,8G | 4G |
| MORNINGGLORY | 2H | 0 | 9G | 3G |
| COCKLEBUR | 1C | 0 | 9H | 3C,5G |
| NUTSEDGE | 0 | 0 | 9G | 0 |
| CRABGRASS | 4C,9G | 0 | 4C,8G | 3C,6G |
| BARNYARDGRASS | 7H | 0 | 9H | 9H |
| WILD OATS | 2C,4G | 0 | 7G | 2G |
| WHEAT | 9G | 7G | 9G | 8G |
| CORN | 3C,9G | 2C,7G | 3G | 0 |
| SOYBEANS | 2C,5G | 2G | 3C,8H | 3C,5G |
| RICE | 9H | 9H | 10E | 9H |
| SORGHUM | 9H | 3C,7G | 10H | 3C,9G |
| CHEATGRASS | 5G | 0 | 8G | 7G |
| SUGARBEETS | 7H | 2H | 9G | 8G |
| VELVETLEAF | 0 | 0 | 5G | 0 |
| GIANT FOXTAIL | 4G | 0 | 3C,8H | 5G |
| BARLEY | 2C,9G | 3G | 9G | 9G |

TABLE A

| | CMPD 7 | | CMPD 8 | |
|---|---|---|---|---|
| RATE=KG/HA | 0.05 | 0.01 | 0.05 | 0.01 |
| POSTEMERGENCE | | | | |
| COTTON | 9C | 9C | 9C | 9C |
| MORNINGGLORY | 10C | 10C | 10C | 10C |
| COCKLEBUR | 10C | 4C,9G | 9C | 9C |
| NUTSEDGE | - | 5C,9G | - | 3C,7G |
| CRABGRASS | 9C | 3C,8G | 4C,9G | 6G |
| BARNYARDGRASS | 9C | 9C | 9C | 9C |
| WILD OATS | 5C,9G | 4C,9G | 2C,9G | 9G |
| WHEAT | 2C,9G | 4C,9G | 9G | 9G |
| CORN | 9G | 3C,9G | 2C,7G | 4G |
| SOYBEANS | 9C | 5C,9G | 3C,9H | 4C,8H |
| RICE | 9C | 9C | 9C | 5C,9G |
| SORGHUM | 9C | 5C,9G | 9C | 5C,9G |
| CHEATGRASS | 9C | 9C | 5C,9G | 5C,9G |
| SUGARBEETS | 9C | 5C,9G | 9C | 9C |
| VELVETLEAF | 10C | 10C | 9C | 5C,9G |
| GIANT FOXTAIL | 9C | 9C | 9C | 5C,9G |
| BARLEY | 4C,9G | 4C,9G | 9G | 2C,7G |

TABLE A

| | CMPD 7 | | CMPD 8 | |
|---|---|---|---|---|
| RATE=KG/HA | 0.05 | 0.01 | 0.05 | 0.01 |
| PREEMERGENCE | | | | |
| COTTON | 3C,9G | 8G | 8G | 3C,7G |
| MORNINGGLORY | 9H | 9G | 9G | 9G |
| COCKLEBUR | 8H | 5G | 3C,6G | 2G |
| NUTSEDGE | 10E | 8G | 10E | 10E |
| CRABGRASS | 4C,9G | 7G | 2C,8G | 3G |
| BARNYARDGRASS | 9H | 9H | 9H | 2C,8G |
| WILD OATS | 4C,8G | 3C,7G | 3C,7G | 3C,7G |
| WHEAT | 3C,9H | 2C,8G | 3C,8G | 3C,8H |
| CORN | 9G | 3C,8H | 3C,8G | 3C,5G |
| SOYBEANS | 9H | 9H | 9H | 3C,7H |
| RICE | 10H | 9H | 10E | 10H |
| SORGHUM | 9H | 4C,9G | 4C,9H | 4C,9G |
| CHEATGRASS | 9H | 8G | 9H | 8G |
| SUGARBEETS | 9G | 9G | 4C,9G | 4C,9G |
| VELVETLEAF | 4C,9G | 8G | 3C,8G | 2C,3G |
| GIANT FOXTAIL | 9H | 4C,9H | 3C,9H | 5G |
| BARLEY | 9H | 2C,8G | 9G | 8G |

TABLE A

| | CMPD 9 | | CMPD 10 | |
|---|---|---|---|---|
| RATE=KG/HA | 0.05 | 0.01 | 0.05 | 0.01 |
| POSTEMERGENCE | | | | |
| COTTON | 9C | 9C | 10C | 10C |
| MORNINGGLORY | 4C,9G | 4C,8H | 10C | 5C,9H |
| COCKLEBUR | 3C,9G | 3C,8H | 10C | 5C,9H |
| NUTSEDGE | 2C,8G | 7G | 10C | 5C,9G |
| CRABGRASS | 5C,9G | 2C,2G | 3C,9G | 2C,4G |
| BARNYARDGRASS | 4C,9G | 3C,8H | 10C | 10C |
| WILD OATS | 4C,9G | 9G | 4C,9G | 3C,9G |
| WHEAT | 3C,9G | 9G | 9C | 5C,9G |
| CORN | 1C,2G | 0 | 4C,9G | 9G |
| SOYBEANS | 0 | 0 | 3C,9G | 2C,7G |
| RICE | 4C,9G | 2C,8G | 9C | 5C,9G |
| SORGHUM | 4C,9G | 3C,8G | 5C,9G | 9G |
| CHEATGRASS | 9G | 6G | 9C | 3C,9G |
| SUGARBEETS | 5C,9G | 4C,8H | 9C | 9C |
| VELVETLEAF | 2C,8H | 4C,7H | 10C | 5C,9H |
| GIANT FOXTAIL | 4C,9G | 2C,7G | 10C | 9C |
| BARLEY | 9G | 6G | 5C,9G | 3C,9G |

TABLE A

| | CMPD 9 | | CMPD 10 | |
|---|---|---|---|---|
| RATE=KG/HA | 0.05 | 0.01 | 0.05 | 0.01 |
| PREEMERGENCE | | | | |
| COTTON | 3G | 2G | 6G | 6G |
| MORNINGGLORY | 2C,8G | 7G | 9H | 9H |
| COCKLEBUR | 3C,8H | 1C,2G | 9H | 9H |
| NUTSEDGE | 5G | 0 | 10E | 10E |
| CRABGRASS | 0 | 0 | 5G | 0 |
| BARNYARDGRASS | 3C,8H | 4G | 9H | 9H |
| WILD OATS | 3C,7G | 2C,5G | 3C,8G | 8G |
| WHEAT | 2C,8H | 8G | 9H | 3C,8H |
| CORN | 0 | 2C,2G | 9G | 3C,8G |
| SOYBEANS | 0 | 0 | 9H | 4C,8H |
| RICE | 9H | 2C,4H | 10H | 9H |
| SORGHUM | 4C,8H | 3C,7H | 4C,9H | 9H |
| CHEATGRASS | 5G | 4G | 9H | 6G |
| SUGARBEETS | 3C,8G | 2C,6G | 3C,9G | 9G |
| VELVETLEAF | 3C,6H | 2C,3H | 3C,9G | 3C,8G |
| GIANT FOXTAIL | 2C,6G | 2G | 4C,9H | 3C,8H |
| BARLEY | 2C,9G | 8G | 3C,9G | 9G |

TABLE A

| | CMPD 11 | | CMPD 12 | |
|---|---|---|---|---|
| RATE=KG/HA | 0.05 | 0.01 | 0.05 | 0.01 |
| POSTEMERGENCE | | | | |
| COTTON | 9C | 6H | 4C,9G | 2C,7G |
| MORNINGGLORY | 5C,9H | 3C,5H | 10C | 4C,9G |
| COCKLEBUR | 4C,8H | 2C,2H | 10C | 4C,9G |
| NUTSEDGE | 4G | 0 | 4C,8G | 4C,7G |
| CRABGRASS | 2G | 0 | 3C,6G | 5G |
| BARNYARDGRASS | 3C,9G | 6G | 5C,9G | 4C,9H |
| WILD OATS | 4C,9G | 3C,9G | 2C,6G | 3G |
| WHEAT | 9G | 9G | 4C,9G | 8G |
| CORN | 3C,9G | 3C,6H | 2C,3G | 0 |
| SOYBEANS | 0 | 0 | 2H | 2G |
| RICE | 4C,9G | 3C,9G | 5C,9G | 4C,8G |
| SORGHUM | 4C,8H | 3C,5G | 4C,9G | 5C,9G |
| CHEATGRASS | 6G | 5G | 5C,9G | 7G |
| SUGARBEETS | 4C,8H | 2C,2H | 10C | 9C |
| VELVETLEAF | 1C | 0 | 2C,8G | 4G |
| GIANT FOXTAIL | 3C,8G | 0 | 3C,7G | 3C,5G |
| BARLEY | 8G | 4G | 4C,9G | 7G |

TABLE A

| | CMPD 11 | | CMPD 12 | |
|---|---|---|---|---|
| RATE=KG/HA | 0.05 | 0.01 | 0.05 | 0.01 |
| PREEMERGENCE | | | | |
| COTTON | 0 | 0 | 3C,8G | 3C,7G |
| MORNINGGLORY | 3C,8G | 2G | 4C,8G | 3C,7G |
| COCKLEBUR | 2C,6G | 2G | 3C,7H | 2C,2H |
| NUTSEDGE | 0 | 0 | 0 | 0 |
| CRABGRASS | 2G | 0 | 3G | 0 |
| BARNYARDGRASS | 6H | 0 | 3C,9H | 3C,7G |
| WILD OATS | 3C,7G | 5G | - | 2C |
| WHEAT | 8G | 6G | 2C,8G | 3C,7G |
| CORN | 3C,8H | 2C,4G | 2C,3G | 0 |
| SOYBEANS | 0 | 0 | 1C | 2G |
| RICE | 9H | 2G | 10E | 4C,9G |
| SORGHUM | 4C,6H | 2C,3G | 4C,9H | 4C,9G |
| CHEATGRASS | 2G | 0 | 8G | 7G |
| SUGARBEETS | 4G | 2G | 9G | 6G |
| VELVETLEAF | 0 | 0 | 5H | 1C,1H |
| GIANT FOXTAIL | 2C,6G | 2G | 2G | 0 |
| BARLEY | 9G | 8G | 2C,8G | 3C,7G |

TABLE A

| | CMPD 13 | |
|---|---|---|
| RATE=KG/HA | 0.05 | 0.01 |
| POSTEMERGENCE | | |
| COTTON | 4C,9G | 7G |
| MORNINGGLORY | 9C | 3C,7G |
| COCKLEBUR | 9C | 4C,9G |
| NUTSEDGE | 3C,6G | 4G |
| CRABGRASS | 4G | 0 |
| BARNYARDGRASS | 4C,9G | 5C,9G |
| WILD OATS | 3C,8G | 3C,6G |
| WHEAT | 3C,9G | 3C,8G |
| CORN | 4C,9G | 3C,8H |
| SOYBEANS | 4C,9G | 3C,7G |
| RICE | 3C,8G | 2C,6G |
| SORGHUM | 3C,8G | 3C,7G |
| CHEATGRASS | 7G | 5G |
| SUGARBEETS | 5C,9G | 4C,8H |
| VELVETLEAF | 5C,9G | 3C,8G |
| GIANT FOXTAIL | 3C,6G | 4G |
| BARLEY | 2C,5G | 2G |

TABLE A

| | CMPD 13 | |
|---|---|---|
| RATE=KG/HA | 0.05 | 0.01 |
| PREEMERGENCE | | |
| COTTON | 5G | 2G |
| MORNINGGLORY | 6H | 3H |
| COCKLEBUR | 3C,6G | 2C,2H |
| NUTSEDGE | 0 | 0 |
| CRABGRASS | 2G | 0 |
| BARNYARDGRASS | 3C,8H | 2G |
| WILD OATS | 3C,6G | 0 |
| WHEAT | 7G | 0 |
| CORN | 3C,8G | 3C,3G |
| SOYBEANS | 3C,6H | 2C,5H |
| RICE | 3C,7G | 2G |
| SORGHUM | 3C,8H | 3C,4G |
| CHEATGRASS | 3C,7G | 0 |
| SUGARBEETS | 6G | 3H |
| VELVETLEAF | 3C,5H | 1H |
| GIANT FOXTAIL | 4G | 0 |
| BARLEY | 3C,8G | 2C |

Test B

Postemergence

Three round pans (25 cm diameter by 12.5 cm deep) were filled with Sassafras sandy loam soil. One pan was planted with nutsedge (Cyperus rotundus) tubers, crabgrass (Digitaria sanguinalis), sicklepod (Cassia obtusifolia), jimsonweed (Datura stramonium), velvetleaf (Abutilon theophrasti), lambsquarters (Chenopodium album), rice (Oryza sativa), and teaweed (Sida spinosa). The second pot was planted with green foxtail (Setaria viridis), cocklebur (Xanthium pensylvanicum), morningglory (Ipomoea hederacea), cotton (Gossypium hirsutum), johnsongrass (Sorghum halepense), barnyardgrass (Echinochloa crus-galli), corn (Zea mays), soybean (Glycine max), and giant foxtail (Setaria faberi). The third pot was planted with wheat (Triticum aestivum), barley (Hordeum vulgare), wild buckwheat (Polygonum convolvulus), downy brome (Bromus tectorum), sugarbeet (Beta vulgaris), wild oat (Avena fatua), common chickweed (Stellaria media), blackgrass (Alopecurus myosuroides), and rape (Brassica napus). The plants were grown for approximately fourteen days, then sprayed postemergence with the chemicals dissolved in a non-phytoxic solvent.

Preemergence

Three round pans (25 cm diameter by 12.5 cm deep) were filled with Sassafras sandy loam soil. One pan was planted with nutsedge tubers, crabgrass, sicklepod, jimsonweed, velvetleaf, lambsquarters, rice, and teaweed. The second pot was planted with green foxtail, cocklebur, morningglory, cotton, johnsongrass, barnyardgrass, corn, soybean, and giant foxtail. The third pot was planted with wheat, barley, wild buckwheat, downy brome, sugarbeet, wild oat, common chickweed, blackgrass, and rape. The three pans were sprayed preemergence with the chemicals dissolved in a non-phytotoxic solvent.

Treated plants and controls were maintained in the greenhouse for approximately 24 days, then all rated plants were compared to controls and visually rated for plant response.

Response ratings are based on a scale of 0 to 100 where 0 indicates no effect, and 100 indicates complete control. A dash (-) response means no test.

Response ratings are contained in Table B.

TABLE B

CMPD 3

| RATE=G/HA | 62 | 16 | 4 | 1 |
|---|---|---|---|---|
| POSTEMERGENCE | | | | |
| GIANT FOXTAIL | 100 | 100 | 100 | 100 |
| VELVETLEAF | 100 | 100 | 100 | 90 |
| SUGARBEETS | 100 | 100 | 100 | 100 |
| CRABGRASS | 100 | 100 | 100 | 60 |
| TEAWEED | 100 | 100 | 90 | 70 |
| JIMSONWEED | 100 | 100 | 100 | 90 |
| RICE | 100 | 100 | 100 | 100 |
| COCKLEBUR | 100 | 90 | 70 | 50 |
| COTTON | 100 | 100 | 70 | 60 |
| SOYBEANS | 100 | 100 | 100 | 90 |
| BARNYARDGRASS | 100 | 100 | 100 | 100 |
| WILD OATS | 100 | 100 | 90 | 70 |
| MORNINGGLORY | 100 | 100 | 100 | 100 |
| WHEAT | 100 | 100 | 100 | 90 |
| SICKLEPOD | 100 | 100 | 100 | 80 |
| JOHNSONGRASS | 100 | 100 | 100 | 100 |
| NUTSEDGE | 100 | 100 | 100 | 100 |
| CORN | 100 | 80 | 20 | 0 |
| WILD BUCKWHEAT | 100 | 100 | 100 | 100 |
| BLACKGRASS | 100 | 100 | 70 | 50 |
| RAPE | 100 | 100 | 100 | 100 |
| BARLEY | 100 | 100 | 80 | 50 |
| GREEN FOXTAIL | 100 | 100 | 100 | 100 |
| LAMBSQUARTER | 100 | 100 | 100 | 100 |
| CHICKWEED | 100 | 100 | 100 | 100 |
| DOWNY BROME | 100 | 100 | 100 | 80 |

TABLE B

CMPD 3

| RATE=G/HA | 62 | 16 | 4 | 1 |
|---|---|---|---|---|
| PREEMERGENCE | | | | |
| GIANT FOXTAIL | 100 | 100 | 80 | 30 |
| VELVETLEAF | 100 | 70 | 60 | 50 |
| SUGARBEETS | 100 | 100 | 100 | 90 |
| CRABGRASS | 100 | 100 | 90 | 60 |
| TEAWEED | 90 | 80 | 70 | 60 |
| JIMSONWEED | 100 | 95 | 90 | 85 |
| RICE | 100 | 100 | 100 | 100 |
| COCKLEBUR | 90 | 80 | 70 | 60 |
| COTTON | 90 | 80 | 70 | 60 |
| SOYBEANS | 90 | 70 | 50 | 0 |
| BARNYARDGRASS | 100 | 90 | 60 | 30 |
| WILD OATS | 80 | 70 | 60 | 30 |
| MORNINGGLORY | 100 | 95 | 90 | 85 |
| WHEAT | 100 | 90 | 80 | 70 |
| SICKLEPOD | 100 | 100 | 100 | 100 |
| JOHNSONGRASS | 100 | 100 | 100 | 70 |
| NUTSEDGE | 100 | 100 | 90 | 80 |
| CORN | 90 | 70 | 30 | 0 |
| WILD BUCKWHEAT | 100 | 100 | 90 | 80 |
| BLACKGRASS | 100 | 100 | 90 | 70 |
| RAPE | 100 | 100 | 100 | 90 |
| BARLEY | 100 | 95 | 90 | 85 |
| GREEN FOXTAIL | 100 | 100 | 100 | 70 |
| LAMBSQUARTER | 100 | 100 | 90 | 80 |
| CHICKWEED | 100 | 95 | 90 | 85 |
| DOWNY BROME | 100 | 100 | 90 | 80 |

TABLE B

CMPD 6

| RATE=G/HA | 62 | 16 | 4 | 1 |
|---|---|---|---|---|
| POSTEMERGENCE | | | | |
| GIANT FOXTAIL | 100 | 100 | 70 | 30 |
| VELVETLEAF | 100 | 80 | 30 | 0 |
| SUGARBEETS | 100 | 100 | 100 | 60 |
| CRABGRASS | 100 | 70 | 60 | 30 |
| TEAWEED | 90 | 80 | 60 | 30 |
| JIMSONWEED | 100 | 100 | 100 | 70 |
| RICE | 100 | 100 | 100 | 30 |
| COCKLEBUR | 100 | 70 | 50 | 30 |
| COTTON | 100 | 70 | 50 | 0 |
| SOYBEANS | 90 | 70 | 50 | 0 |
| BARNYARDGRASS | 100 | 100 | 100 | 90 |
| WILD OATS | 70 | 50 | 30 | 0 |
| MORNINGGLORY | 100 | 100 | 100 | 70 |
| WHEAT | 70 | 50 | 30 | 0 |
| SICKLEPOD | 100 | 100 | 50 | 0 |
| JOHNSONGRASS | 100 | 100 | 100 | 100 |
| NUTSEDGE | 100 | 80 | 60 | 50 |
| CORN | 0 | 0 | 0 | 0 |
| WILD BUCKWHEAT | 100 | 100 | 90 | 70 |
| BLACKGRASS | 90 | 70 | 50 | 30 |
| RAPE | 100 | 100 | 100 | 100 |
| BARLEY | 90 | 60 | 30 | 0 |
| GREEN FOXTAIL | 100 | 100 | 60 | 40 |
| LAMBSQUARTER | 100 | 100 | 100 | 100 |
| CHICKWEED | 100 | 100 | 50 | 30 |
| DOWNY BROME | 90 | 80 | 60 | 30 |

TABLE B

CMPD 6

| RATE=G/HA | 62 | 16 | 4 | 1 |
|---|---|---|---|---|
| PREEMERGENCE | | | | |
| GIANT FOXTAIL | 100 | 80 | 30 | 0 |
| VELVETLEAF | 90 | 60 | 30 | 0 |
| SUGARBEETS | 100 | 100 | 100 | 100 |
| CRABGRASS | 90 | 80 | 60 | 30 |
| TEAWEED | 95 | 90 | 80 | 70 |
| JIMSONWEED | 100 | 90 | 60 | 30 |
| RICE | 100 | 100 | 100 | 100 |
| COCKLEBUR | 90 | 80 | 70 | 50 |
| COTTON | 90 | 70 | 30 | 0 |
| SOYBEANS | 80 | 50 | 0 | 0 |
| BARNYARDGRASS | 100 | 70 | 30 | 0 |
| WILD OATS | 80 | 50 | 0 | 0 |
| MORNINGGLORY | 100 | 90 | 80 | 70 |
| WHEAT | 90 | 70 | 50 | 0 |
| SICKLEPOD | 100 | 100 | 100 | 100 |
| JOHNSONGRASS | 100 | 100 | 70 | 50 |
| NUTSEDGE | 100 | 90 | 30 | 0 |
| CORN | 30 | 0 | 0 | 0 |
| WILD BUCKWHEAT | 100 | 100 | 90 | 70 |
| BLACKGRASS | 90 | 80 | 70 | 60 |
| RAPE | 100 | 100 | 100 | 90 |
| BARLEY | 100 | 100 | 80 | 50 |
| GREEN FOXTAIL | 100 | 90 | 70 | 30 |
| LAMBSQUARTER | 100 | 90 | 70 | 50 |
| CHICKWEED | 90 | 70 | 30 | 0 |
| DOWNY BROME | 100 | 100 | 90 | 80 |

CMPD 7

| RATE=G/HA | 62 | 16 | 4 | 1 |
|---|---|---|---|---|
| POSTEMERGENCE | | | | |
| GIANT FOXTAIL | 100 | 100 | 100 | 70 |
| VELVETLEAF | 100 | 100 | 80 | 50 |
| SUGARBEETS | 100 | 100 | 100 | 100 |
| CRABGRASS | 100 | 100 | 80 | 40 |
| TEAWEED | 100 | 90 | 30 | 20 |
| JIMSONWEED | 100 | 100 | 90 | 70 |
| RICE | 100 | 100 | 100 | 90 |
| COCKLEBUR | 100 | 80 | 20 | 0 |
| COTTON | 100 | 100 | 90 | 50 |
| SOYBEANS | 100 | 100 | 90 | 70 |
| BARNYARDGRASS | 100 | 100 | 100 | 100 |
| WILD OATS | 100 | 100 | 100 | 90 |
| MORNINGGLORY | 100 | 100 | 90 | 30 |
| WHEAT | 100 | 100 | 100 | 80 |
| SICKLEPOD | 100 | 100 | 50 | 30 |
| JOHNSONGRASS | 100 | 100 | 90 | 60 |
| NUTSEDGE | 100 | 90 | 70 | 20 |
| CORN | 100 | 90 | 90 | 40 |
| WILD BUCKWHEAT | 100 | 100 | 100 | 70 |
| BLACKGRASS | 100 | 100 | 100 | 90 |
| RAPE | 100 | 100 | 100 | 100 |
| BARLEY | 100 | 100 | 100 | 90 |
| GREEN FOXTAIL | 100 | 100 | 70 | 70 |
| LAMBSQUARTER | 100 | 100 | 80 | 80 |
| CHICKWEED | 100 | 100 | 100 | 100 |
| DOWNY BROME | 100 | 100 | 100 | 100 |

TABLE B

CMPD 7

| RATE=G/HA | 62 | 16 | 4 | 1 |
|---|---|---|---|---|
| PREEMERGENCE | | | | |
| GIANT FOXTAIL | 100 | 100 | 90 | 30 |
| VELVETLEAF | 100 | 100 | 80 | 40 |
| SUGARBEETS | 100 | 90 | 90 | 40 |
| CRABGRASS | 100 | 100 | 100 | 40 |
| TEAWEED | 90 | 90 | 80 | 70 |
| JIMSONWEED | 100 | 90 | 80 | 60 |
| RICE | 100 | 100 | 90 | 80 |
| COCKLEBUR | 80 | 70 | 40 | 0 |
| COTTON | 90 | 90 | 80 | 40 |
| SOYBEANS | 90 | 90 | 40 | 10 |
| BARNYARDGRASS | 100 | 100 | 50 | 0 |
| WILD OATS | 80 | 70 | 40 | 0 |
| MORNINGGLORY | 100 | 100 | 80 | 70 |
| WHEAT | 80 | 70 | 50 | 10 |
| SICKLEPOD | 100 | 100 | 100 | 40 |
| JOHNSONGRASS | 100 | 90 | 90 | 50 |
| NUTSEDGE | 100 | 100 | 50 | 20 |
| CORN | 100 | 70 | 20 | 20 |
| WILD BUCKWHEAT | 100 | 90 | 90 | 40 |
| BLACKGRASS | 100 | 90 | 60 | 40 |
| RAPE | 100 | 100 | 60 | 50 |
| BARLEY | 80 | 80 | 50 | 10 |
| GREEN FOXTAIL | 100 | 100 | 100 | 50 |
| LAMBSQUARTER | 100 | 100 | 100 | 90 |
| CHICKWEED | 100 | 100 | 100 | 90 |
| DOWNY BROME | 100 | 100 | 50 | 30 |

TABLE B

| CMPD 8 | | | | |
|---|---|---|---|---|
| RATE=G/HA | 62 | 16 | 4 | 1 |
| POSTEMERGENCE | | | | |
| GIANT FOXTAIL | 100 | 90 | 40 | 0 |
| VELVETLEAF | 100 | 80 | 40 | 20 |
| SUGARBEETS | 100 | 100 | 100 | 90 |
| CRABGRASS | 100 | 80 | 50 | 20 |
| TEAWEED | 100 | 100 | 50 | 20 |
| JIMSONWEED | 100 | 100 | 90 | 60 |
| RICE | 100 | 100 | 80 | 30 |
| COCKLEBUR | 100 | 50 | 20 | 0 |
| COTTON | 100 | 90 | 70 | 30 |
| SOYBEANS | 100 | 100 | 80 | 40 |
| BARNYARDGRASS | 100 | 100 | 100 | 90 |
| WILD OATS | 100 | 80 | 70 | 40 |
| MORNINGGLORY | 100 | 100 | 80 | 40 |
| WHEAT | 90 | 70 | 50 | 30 |
| SICKLEPOD | 100 | 90 | 50 | 30 |
| JOHNSONGRASS | 100 | 100 | 100 | 40 |
| NUTSEDGE | 100 | 100 | 60 | 20 |
| CORN | 70 | 20 | 0 | 0 |
| WILD BUCKWHEAT | 100 | 90 | 70 | 60 |
| BLACKGRASS | 100 | 100 | 80 | 60 |
| RAPE | 100 | 100 | 100 | 80 |
| BARLEY | 100 | 80 | 70 | 40 |
| GREEN FOXTAIL | 100 | 100 | 80 | 50 |
| LAMBSQUARTER | 100 | 90 | 90 | 30 |
| CHICKWEED | 100 | 100 | 70 | 50 |
| DOWNY BROME | 100 | 100 | 60 | 30 |

TABLE B

CMPD 8

| RATE=G/HA | 62 | 16 | 4 | 1 |
|---|---|---|---|---|
| PREEMERGENCE | | | | |
| GIANT FOXTAIL | 100 | 90 | 40 | 10 |
| VELVETLEAF | 100 | 80 | 50 | 20 |
| SUGARBEETS | 100 | 90 | 60 | 40 |
| CRABGRASS | 100 | 90 | 90 | 50 |
| TEAWEED | 90 | 90 | 40 | 20 |
| JIMSONWEED | 100 | 100 | 70 | 40 |
| RICE | 100 | 100 | 90 | 40 |
| COCKLEBUR | 100 | 80 | 30 | 0 |
| COTTON | 90 | 90 | 50 | 20 |
| SOYBEANS | 70 | 50 | 20 | 10 |
| BARNYARDGRASS | 100 | 100 | 40 | 10 |
| WILD OATS | 90 | 50 | 10 | 0 |
| MORNINGGLORY | 90 | 90 | 50 | 40 |
| WHEAT | 80 | 70 | 50 | 10 |
| SICKLEPOD | 100 | 90 | 70 | - |
| JOHNSONGRASS | 100 | 100 | 100 | 70 |
| NUTSEDGE | 100 | 40 | 40 | 0 |
| CORN | 30 | 30 | 10 | 0 |
| WILD BUCKWHEAT | 100 | 80 | 70 | 60 |
| BLACKGRASS | 100 | 90 | 70 | 60 |
| RAPE | 100 | 80 | 60 | 50 |
| BARLEY | 90 | 50 | 40 | 30 |
| GREEN FOXTAIL | 100 | 100 | 80 | 30 |
| LAMBSQUARTER | 100 | 90 | 90 | - |
| CHICKWEED | 100 | 100 | 90 | 60 |
| DOWNY BROME | 100 | 80 | 60 | 20 |

TABLE B

CMPD 9

| RATE=G/HA | 250 | 62 | 16 | 4 |
|---|---|---|---|---|
| POSTEMERGENCE | | | | |
| GIANT FOXTAIL | 70 | 50 | 30 | 0 |
| VELVETLEAF | 100 | 100 | 100 | 70 |
| SUGARBEETS | 100 | 100 | 90 | 80 |
| CRABGRASS | 60 | 50 | 40 | 30 |
| TEAWEED | 90 | 80 | 70 | 50 |
| JIMSONWEED | 100 | 90 | 80 | 60 |
| RICE | 100 | 90 | 60 | 30 |
| COCKLEBUR | 70 | 60 | 50 | 30 |
| COTTON | 70 | 30 | – | 0 |
| SOYBEANS | 50 | 30 | 0 | 0 |
| BARNYARDGRASS | 100 | 80 | 60 | 30 |
| WILD OATS | 90 | 80 | 70 | 60 |
| MORNINGGLORY | 90 | 70 | 60 | 50 |
| WHEAT | 100 | 100 | 60 | 50 |
| SICKLEPOD | 60 | 50 | 40 | 30 |
| JOHNSONGRASS | 90 | 80 | 70 | 60 |
| NUTSEDGE | 100 | 100 | 70 | 50 |
| CORN | 0 | 0 | 0 | 0 |
| WILD BUCKWHEAT | 80 | 60 | 50 | 0 |
| BLACKGRASS | 100 | 100 | 90 | 70 |
| RAPE | 100 | 100 | 100 | 80 |
| BARLEY | 90 | 60 | 50 | 40 |
| GREEN FOXTAIL | 80 | 50 | 30 | 0 |
| LAMBSQUARTER | 100 | 80 | 70 | 50 |
| CHICKWEED | 100 | 100 | 70 | 60 |
| DOWNY BROME | 100 | 70 | 50 | 30 |

TABLE B

CMPD 9

| RATE=G/HA | 250 | 62 | 16 | 4 |
|---|---|---|---|---|
| PREEMERGENCE | | | | |
| GIANT FOXTAIL | 90 | 70 | 30 | 0 |
| VELVETLEAF | 90 | 70 | 30 | 0 |
| SUGARBEETS | 70 | 50 | 30 | 0 |
| CRABGRASS | 50 | 30 | 0 | 0 |
| TEAWEED | 90 | 70 | 50 | 30 |
| JIMSONWEED | 70 | 50 | 30 | 0 |
| RICE | 100 | 100 | 70 | 30 |
| COCKLEBUR | 80 | 50 | 30 | 0 |
| COTTON | 50 | 30 | 0 | 0 |
| SOYBEANS | 0 | 0 | 0 | 0 |
| BARNYARDGRASS | 100 | 80 | 60 | 50 |
| WILD OATS | 70 | 30 | 0 | 0 |
| MORNINGGLORY | 70 | 50 | 30 | 0 |
| WHEAT | 70 | 50 | 30 | 0 |
| SICKLEPOD | 70 | 50 | 30 | 0 |
| JOHNSONGRASS | 90 | 80 | 60 | 30 |
| NUTSEDGE | 90 | 60 | 30 | 0 |
| CORN | 0 | 0 | 0 | 0 |
| WILD BUCKWHEAT | 100 | 95 | 90 | 85 |
| BLACKGRASS | 90 | 80 | 70 | 60 |
| RAPE | 100 | 90 | 70 | 0 |
| BARLEY | 80 | 70 | 50 | 30 |
| GREEN FOXTAIL | 90 | 70 | 30 | 0 |
| LAMBSQUARTER | 100 | 100 | 70 | 50 |
| CHICKWEED | 0 | 0 | 0 | 0 |
| DOWNY BROME | 70 | 50 | 30 | 0 |

TABLE B

CMPD 10

| RATE=G/HA | 62 | 16 | 4 | 1 |
|---|---|---|---|---|
| POSTEMERGENCE | | | | |
| GIANT FOXTAIL | 100 | 80 | 50 | 30 |
| VELVETLEAF | 100 | 100 | 100 | 80 |
| SUGARBEETS | 100 | 100 | 100 | 100 |
| CRABGRASS | 80 | 50 | 40 | 30 |
| TEAWEED | 100 | 90 | 60 | 30 |
| JIMSONWEED | 100 | 90 | 60 | 40 |
| RICE | 90 | 80 | 70 | 60 |
| COCKLEBUR | 100 | 100 | 70 | 30 |
| COTTON | 70 | 40 | 30 | 0 |
| SOYBEANS | 100 | 80 | 60 | 0 |
| BARNYARDGRASS | 100 | 100 | 70 | 60 |
| WILD OATS | 100 | 90 | 60 | 30 |
| MORNINGGLORY | 100 | 100 | 90 | 50 |
| WHEAT | 100 | 100 | 80 | 60 |
| SICKLEPOD | 90 | 60 | 30 | 0 |
| JOHNSONGRASS | 100 | 100 | 100 | 70 |
| NUTSEDGE | - | - | 70 | 50 |
| CORN | 80 | 70 | 0 | 0 |
| WILD BUCKWHEAT | 100 | 100 | 70 | 50 |
| BLACKGRASS | 100 | 100 | 100 | 100 |
| RAPE | 100 | 100 | 100 | 100 |
| BARLEY | 90 | 70 | 50 | 30 |
| GREEN FOXTAIL | 100 | 80 | 50 | 30 |
| LAMBSQUARTER | 100 | 100 | 90 | 60 |
| CHICKWEED | 100 | 100 | 100 | 100 |
| DOWNY BROME | 100 | 100 | 80 | 50 |

TABLE B

CMPD 10

| RATE=G/HA | 62 | 16 | 4 | 1 |
|---|---|---|---|---|
| PREEMERGENCE | | | | |
| GIANT FOXTAIL | 100 | 100 | 80 | 30 |
| VELVETLEAF | 100 | 90 | 80 | 70 |
| SUGARBEETS | 90 | 80 | 70 | 30 |
| CRABGRASS | 100 | 90 | 60 | 30 |
| TEAWEED | 90 | 80 | 70 | 30 |
| JIMSONWEED | 90 | 80 | 70 | 30 |
| RICE | 100 | 100 | 100 | 90 |
| COCKLEBUR | 90 | 80 | 70 | 40 |
| COTTON | 70 | 50 | 30 | 0 |
| SOYBEANS | 70 | 50 | 30 | 0 |
| BARNYARDGRASS | 100 | 100 | 100 | 50 |
| WILD OATS | 60 | 30 | 0 | 0 |
| MORNINGGLORY | 90 | 80 | 50 | 30 |
| WHEAT | 80 | 60 | 30 | 0 |
| SICKLEPOD | 90 | 70 | 50 | 30 |
| JOHNSONGRASS | 90 | 80 | 70 | 50 |
| NUTSEDGE | 100 | 100 | 95 | 90 |
| CORN | 100 | 90 | 60 | 0 |
| WILD BUCKWHEAT | 95 | 90 | 85 | 80 |
| BLACKGRASS | 100 | 90 | 80 | 50 |
| RAPE | 100 | 100 | 100 | 80 |
| BARLEY | 80 | 70 | 60 | 50 |
| GREEN FOXTAIL | 100 | 90 | 70 | 50 |
| LAMBSQUARTER | 100 | 90 | 80 | 70 |
| CHICKWEED | 100 | 100 | 90 | 50 |
| DOWNY BROME | 90 | 60 | 30 | 0 |

TABLE B

CMPD 11

| RATE=G/HA | 250 | 62 | 16 | 4 |
|---|---|---|---|---|
| POSTEMERGENCE | | | | |
| GIANT FOXTAIL | 70 | 50 | 30 | 0 |
| VELVETLEAF | 70 | 30 | 0 | 0 |
| SUGARBEETS | 100 | 70 | 0 | 0 |
| CRABGRASS | 70 | 60 | 50 | 40 |
| TEAWEED | 70 | 50 | 30 | 0 |
| JIMSONWEED | 100 | 80 | 50 | 40 |
| RICE | 100 | 90 | 60 | 30 |
| COCKLEBUR | 50 | 30 | 0 | 0 |
| COTTON | 30 | 20 | 0 | 0 |
| SOYBEANS | 40 | 30 | 0 | 0 |
| BARNYARDGRASS | 90 | 70 | 50 | 30 |
| WILD OATS | 100 | 90 | 60 | 50 |
| MORNINGGLORY | 70 | 30 | 0 | 0 |
| WHEAT | 100 | 100 | 70 | 60 |
| SICKLEPOD | 60 | 30 | 0 | 0 |
| JOHNSONGRASS | 70 | 60 | 50 | 30 |
| NUTSEDGE | 50 | 30 | 0 | 0 |
| CORN | 80 | 50 | 30 | 0 |
| WILD BUCKWHEAT | 90 | 60 | 30 | 0 |
| BLACKGRASS | 100 | 100 | 70 | 60 |
| RAPE | 100 | 100 | 100 | 90 |
| BARLEY | 100 | 70 | 60 | 50 |
| GREEN FOXTAIL | 80 | 70 | 50 | 30 |
| LAMBSQUARTER | 70 | 60 | 50 | 30 |
| CHICKWEED | 100 | 90 | 60 | 30 |
| DOWNY BROME | 100 | 100 | 50 | 30 |

TABLE B

CMPD 11

| RATE=G/HA | 250 | 62 | 16 | 4 |
|---|---|---|---|---|
| PREEMERGENCE | | | | |
| GIANT FOXTAIL | 70 | 50 | 30 | 0 |
| VELVETLEAF | 70 | 60 | 30 | 0 |
| SUGARBEETS | 70 | 50 | 0 | 0 |
| CRABGRASS | 100 | 90 | 60 | 30 |
| TEAWEED | 70 | 50 | 30 | 0 |
| JIMSONWEED | 50 | 30 | 0 | 0 |
| RICE | 100 | 90 | 60 | 30 |
| COCKLEBUR | 50 | 30 | 0 | 0 |
| COTTON | 30 | 20 | 0 | 0 |
| SOYBEANS | 0 | 0 | 0 | 0 |
| BARNYARDGRASS | 100 | 60 | 30 | 0 |
| WILD OATS | 70 | 50 | 30 | 0 |
| MORNINGGLORY | 80 | 40 | 0 | 0 |
| WHEAT | 80 | 30 | 0 | 0 |
| SICKLEPOD | 90 | 70 | 50 | 40 |
| JOHNSONGRASS | 90 | 80 | 50 | 30 |
| NUTSEDGE | 50 | 30 | 0 | 0 |
| CORN | 90 | 80 | 0 | 0 |
| WILD BUCKWHEAT | 95 | 90 | 80 | 70 |
| BLACKGRASS | 90 | 80 | 70 | 50 |
| RAPE | 70 | 30 | 0 | 0 |
| BARLEY | 80 | 50 | 0 | 0 |
| GREEN FOXTAIL | 90 | 60 | 30 | 0 |
| LAMBSQUARTER | 90 | 70 | 50 | 30 |
| CHICKWEED | 70 | 60 | 50 | 30 |
| DOWNY BROME | 80 | 50 | 0 | 0 |

Test C

The Corn and Sorghum Herbicide Test included the following species in both the preemergence and postemergence evaluations:

| Category | SPECIES Common Name | Scientific Name |
|---|---|---|
| Crops | Corn | *Zea mays* |
| | Soybean | *Glycine max* |
| | Sorghum | *Sorghum bicolor* |
| Grasses | Green foxtail | *Setaria viridis* |
| | Giant foxtail | *Setaria faberii* |
| | Johnsongrass | *Sorghum halepense* |
| | Barnyardgrass | *Echinochloa crus-galli* |
| | Fall panicum | *Panicum dichotomiflorum* |
| | Crabgrass | *Digitaria sanguinalis* |
| | Nutsedge | *Cyperus rotundus* |
| Broadleaves | Cocklebur | *Xanthium pensylvanicum* |
| | Morningglory | *Ipomoea hederacea* |
| | Velvetleaf | *Abutilon theophrasti* |
| | Jimsonweed | *Datura stramonium* |
| | Lambsquarters | *Chenopodium album* |
| | Pigweed | *Amaranthus retroflexus* |
| | Smartweed | *Polygonum persicaris* |

Postemergence

Postemergence plantings were grown in Sassafras sandy loam soil. Corn and soybeans were grown in separate 25-cm diameter containers. Sorghum and the seven grass weed species were grown in two 18-cm diameter containers, 4 species per container. The seven broadleaf weed species were also grown in two 18-cm diameter containers, 4 species in one container, 3 species in the second container. One additional planting of corn in an 18-cm diameter container was made. The soil surface of this additional container of corn was covered with the absorbent, perlite, before spray treatment so that test chemicals would enter the plant only via the foliage. The plants were grown 10-21 days, dependent upon the species and then sprayed postemergence with the test chemicals dissolved in a nonphytotoxic solvent.

Preemergence

Preemergence plantings were grown in fertilized Tama silt loam soil. These plantings are identical to those described in the postemergence section, with the exception of the corn planting having perlite covering the soil surface. These plantings were made the day of or the day before spraying the test chemicals dissolved in a nonphytotoxic solvent.

Evaluation

Treated plants and controls were maintained in the greenhouse for 2 to 4 weeks. Visual plant response ratings were made on a percentage scale of 0 to 100 in comprison with a control where 0 indicates no injury, and 100 indicates death. Response ratings are contained in Table C.

TABLE C

CMPD 1

| RATE GM/HA | 2 | 4 | 8 | 16 | 32 | 64 | 125 |
|---|---|---|---|---|---|---|---|
| POSTEMERGENCE | | | | | | | |
| CORN | 0 | 0 | 0 | 0 | 0 | 0 | 25 |
| SOYBEANS | 55 | 55 | 85 | 100 | 100 | 100 | 100 |
| GREEN FOXTAIL | 20 | 20 | 35 | 55 | 75 | 85 | 95 |
| GIANT FOXTAIL | 0 | 0 | 30 | 45 | 70 | 85 | 95 |
| FALL PANICUM | 0 | 0 | 35 | 45 | 65 | 80 | 95 |
| CRABGRASS | 0 | 0 | 0 | 0 | 25 | 35 | 50 |
| BARNYARDGRASS | 35 | 25 | 50 | 85 | 100 | 100 | 100 |
| JOHNSONGRASS | 40 | 35 | 60 | 100 | 100 | 100 | 100 |
| SORGHUM | 35 | 30 | 50 | 75 | 85 | 100 | 100 |
| NUTSEDGE | 0 | 30 | 45 | 70 | 85 | 100 | 100 |
| VELVETLEAF | 0 | 20 | 45 | 65 | 70 | 85 | 95 |
| COCKLEBUR | 0 | 0 | 20 | 35 | 50 | 80 | 100 |
| SMARTWEED | 30 | 0 | 25 | 55 | 80 | 90 | 95 |
| LAMBSQUARTER | 40 | 20 | 50 | 75 | 90 | 100 | 100 |
| PIGWEED | 75 | 60 | 90 | 100 | 100 | 100 | 100 |
| MORNINGLORY | 0 | 20 | 40 | 60 | 65 | 85 | 100 |
| JIMSONWEED | 25 | 40 | 70 | 95 | 100 | 100 | 100 |
| PERLITE CORN[1] | 0 | 0 | 0 | 0 | 0 | 0 | 20 |

[1]Denotes results using soil covered with perlite at time of treatment as noted in the test description.

TABLE C

CMPD 3

| RATE GM/HA | 2 | 4 | 8 | 16 | 32 | 64 |
|---|---|---|---|---|---|---|
| POSTEMERGENCE | | | | | | |
| CORN | 0 | 25 | 50 | 75 | 85 | 100 |
| SOYBEANS | 100 | 100 | 100 | 100 | 100 | 100 |
| GREEN FOXTAIL | 85 | 100 | 100 | 100 | 100 | 100 |
| GIANT FOXTAIL | 85 | 100 | 100 | 100 | 100 | 100 |
| FALL PANICUM | 80 | 100 | 100 | 100 | 100 | 100 |
| LARGE CRABGRASS | 35 | 65 | 90 | 100 | 100 | 100 |
| BARNYARDGRASS | 90 | 100 | 100 | 100 | 100 | 100 |
| JOHNSONGRASS | 100 | 100 | 100 | 100 | 100 | 100 |
| SORGHUM | 95 | 100 | 100 | 100 | 100 | 100 |
| NUTSEDGE | 75 | 85 | 100 | 100 | 100 | 100 |
| VELVETLEAF | 65 | 85 | 90 | 100 | 100 | 100 |
| COCKLEBUR | 20 | 35 | 55 | 65 | 70 | 85 |
| SMARTWEED | 60 | 75 | 90 | 100 | 100 | 100 |
| LAMBSQUARTER | 85 | 100 | 100 | 100 | 100 | 100 |
| PIGWEED | 100 | 100 | 100 | 100 | 100 | 100 |
| MORNINGLORY | 75 | 90 | 100 | 100 | 100 | 100 |
| JIMSONWEED | 60 | 80 | 95 | 100 | 100 | 100 |
| PERLITE CORN[1] | 0 | 0 | 30 | 55 | 65 | 75 |

TABLE C

CMPD 3

| RATE GM/HA | 16 | 32 | 64 | 125 | 250 |
|---|---|---|---|---|---|
| PREEMERGENCE | | | | | |
| CORN | 0 | 0 | 35 | 45 | 70 |
| SOYBEANS | 35 | 65 | 85 | 100 | 100 |
| GREEN FOXTAIL | 45 | 75 | 100 | 100 | 100 |
| GIANT FOXTAIL | 30 | 55 | 80 | 80 | 100 |
| FALL PANICUM | 50 | 80 | 95 | 100 | 100 |
| CRABGRASS | 35 | 50 | 90 | 100 | 100 |
| BARNYARDGRASS | 45 | 80 | 100 | 100 | 100 |
| JOHNSONGRASS | 75 | 100 | 100 | 100 | 100 |
| SORGHUM | 55 | 75 | 95 | 100 | 100 |
| NUTSEDGE | 30 | 75 | 90 | 100 | 100 |
| VELVETLEAF | 60 | 75 | 85 | 95 | 100 |
| COCKLEBUR | 0 | 25 | 45 | 65 | 80 |
| SMARTWEED | 80 | 100 | 100 | 100 | 100 |
| LAMBSQUARTER | 80 | 100 | 100 | 100 | 100 |
| PIGWEED | 90 | 100 | 100 | 100 | 100 |
| MORNINGLORY | 35 | 55 | 75 | 85 | 95 |
| JIMSONWEED | 25 | 50 | 70 | 95 | 100 |

[1]Denotes results using soil covered with perlite at time of treatment as noted in the test description.

TABLE C

CMPD 6

| RATE GM/HA | 2 | 4 | 8 | 16 | 32 | 64 | 125 |
|---|---|---|---|---|---|---|---|
| POSTEMERGENCE | | | | | | | |
| CORN | 0 | 0 | 0 | 0 | 0 | 0 | 25 |
| SOYBEANS | 20 | 45 | 55 | 70 | 85 | 100 | 100 |
| GREEN FOXTAIL | 20 | 25 | 40 | 75 | 90 | 100 | 100 |
| GIANT FOXTAIL | 0 | 0 | 0 | 40 | 65 | 90 | 100 |
| FALL PANICUM | 20 | 0 | 25 | 50 | 75 | 90 | 100 |
| CRABGRASS | 0 | 0 | 0 | 25 | 45 | 60 | 75 |
| BARNYARDGRASS | 75 | 50 | 95 | 100 | 100 | 100 | 100 |
| JOHNSONGRASS | 80 | 95 | 100 | 100 | 100 | 100 | 100 |
| SORGHUM | 70 | 90 | 100 | 100 | 100 | 100 | 100 |
| NUTSEDGE | 25 | 0 | 35 | 75 | 100 | 100 | 100 |
| VELVETLEAF | 0 | 0 | 0 | 25 | 40 | 70 | 90 |
| COCKLEBUR | 25 | 25 | 50 | 65 | 90 | 100 | 100 |
| SMARTWEED | 20 | 0 | 25 | 40 | 80 | 90 | 95 |
| LAMBSQUARTER | 35 | 20 | 40 | 75 | 95 | 100 | 100 |
| PIGWEED | 90 | 65 | 95 | 100 | 100 | 100 | 100 |
| MORNINGLORY | 20 | 0 | 35 | 45 | 80 | 95 | 95 |
| JIMSONWEED | 45 | 35 | 65 | 85 | 100 | 100 | 100 |
| PERLITE CORN[1] | 0 | 0 | 0 | 0 | 0 | 0 | 20 |

TABLE C

CMPD 6

| RATE GM/HA | 16 | 32 | 64 | 125 | 250 |
|---|---|---|---|---|---|
| PREEMERGENCE | | | | | |
| CORN | 0 | 0 | 0 | 0 | 30 |
| SOYBEANS | 0 | 0 | 20 | 35 | 65 |
| GREEN FOXTAIL | 20 | 40 | 70 | 85 | 100 |
| GIANT FOXTAIL | 20 | 40 | 70 | 85 | 100 |
| FALL PANICUM | 0 | 20 | 65 | 90 | 100 |
| CRABGRASS | 0 | 20 | 50 | 75 | 90 |
| BARNYARDGRASS | 25 | 35 | 75 | 100 | 100 |
| JOHNSONGRASS | 70 | 90 | 100 | 100 | 100 |
| SORGHUM | 70 | 85 | 95 | 100 | 100 |
| NUTSEDGE | 25 | 60 | 70 | 85 | 100 |
| VELVETLEAF | 0 | 0 | 0 | 20 | 45 |
| COCKLEBUR | 0 | 0 | 0 | 0 | 30 |
| SMARTWEED | 25 | 55 | 85 | 100 | 100 |
| LAMBSQUARTER | 35 | 60 | 85 | 100 | 100 |
| PIGWEED | 40 | 65 | 95 | 100 | 100 |
| MORNINGLORY | 0 | 0 | 0 | 0 | 50 |
| JIMSONWEED | 25 | 35 | 65 | 90 | 100 |

[1]Denotes results using soil covered with perlite at time of treatment as noted in the test description.

TABLE C

CMPD 8

| RATE GM/HA | 2 | 4 | 8 | 16 | 32 | 64 |
|---|---|---|---|---|---|---|
| POSTEMERGENCE | | | | | | |
| CORN | 0 | 0 | 0 | 20 | 40 | 65 |
| SOYBEANS | 30 | 50 | 70 | 90 | 100 | 100 |
| GREEN FOXTAIL | 25 | 65 | 85 | 95 | 100 | 100 |
| GIANT FOXTAIL | 25 | 45 | 70 | 85 | 90 | 100 |
| FALL PANICUM | 20 | 45 | 65 | 85 | 100 | 100 |
| CRABGRASS | 0 | 35 | 55 | 80 | 95 | 100 |
| BARNYARDGRASS | 60 | 90 | 100 | 100 | 100 | 100 |
| JOHNSONGRASS | 65 | 100 | 100 | 100 | 100 | 100 |
| SORGHUM | 60 | 95 | 100 | 100 | 100 | 100 |
| NUTSEDGE | 20 | 50 | 80 | 95 | 100 | 100 |
| VELVETLEAF | 0 | 0 | 20 | 40 | 65 | 80 |
| COCKLEBUR | 0 | 25 | 45 | 85 | 95 | 95 |
| SMARTWEED | 35 | 45 | 65 | 75 | 85 | 90 |
| LAMBSQUARTER | 40 | 85 | 95 | 100 | 100 | 100 |
| PIGWEED | 75 | 100 | 100 | 100 | 100 | 100 |
| MORNINGLORY | 0 | 0 | 25 | 60 | 75 | 85 |
| JIMSONWEED | 35 | 80 | 100 | 100 | 100 | 100 |
| PERLITE CORN[1] | 0 | 0 | 0 | 20 | 35 | 60 |

TABLE C

CMPD 8

| RATE GM/HA | 16 | 32 | 64 | 125 | 250 |
|---|---|---|---|---|---|
| PREEMERGENCE | | | | | |
| CORN | 0 | 0 | 0 | 25 | 40 |
| SOYBEANS | 0 | 20 | 50 | 70 | 85 |
| GREEN FOXTAIL | 40 | 80 | 95 | 100 | 100 |
| GIANT FOXTAIL | 0 | 25 | 65 | 100 | 100 |
| FALL PANICUM | 45 | 75 | 95 | 100 | 100 |
| CRABGRASS | 25 | 55 | 85 | 100 | 100 |
| BARNYARDGRASS | 50 | 85 | 100 | 100 | 100 |
| JOHNSONGRASS | 70 | 95 | 100 | 100 | 100 |
| SORGHUM | 50 | 80 | 90 | 100 | 100 |
| NUTSEDGE | 0 | 35 | 65 | 85 | 100 |
| VELVETLEAF | 0 | 0 | 0 | 40 | 55 |
| COCKLEBUR | 0 | 0 | 0 | 20 | 40 |
| SMARTWEED | 40 | 70 | 95 | 100 | 100 |
| LAMBSQUARTER | 60 | 90 | 100 | 100 | 100 |
| PIGWEED | 65 | 90 | 100 | 100 | 100 |
| MORNINGLORY | 0 | 0 | 0 | 25 | 40 |
| JIMSONWEED | 0 | 0 | 20 | 40 | 70 |

[1]Denotes results using soil covered with perlite at time of treatment as noted in the test description.

Test D

Seeds of downy brome (Bromus tectorum), kochia (Kochia scoparia), optionally Russian thistle (Salsoa kali), wild oats (Avena fatua), field bindweed (Convolvulus arvensis), rye (Secale cereale), green foxtail (Setaria viridis) and winter wheat (Triticum aestivum) were placed in 26-cm plastic pans containing a pasteurized sandy loam soil (pH 6.5, 1% organic matter). Plantings were maintained in the greenhouse for 28 days at which time the postemergence treatments were applied using a nonphytotoxic solvent as the carrier for the herbicide. The preemergence segment of the test was seeded immediately before herbicide application using seeds of downy brome (Bromus tectorum), kochia (Kochia scoparia), optionally Russian thistle (Salsoa kali), wild oats (Avena fatua), winter wheat (Triticum aestivum), field bindweed (Convolvulus arvensis), rye (Secale cereale), green foxtail (Setaria viridis), jointed goatgrass (Aegilops cylindrica), wild buckwheat (Polygonium convolvus), pigweed (Amaranthus retroflexus) and lambsquarters (Chenopodium album). A separate pot was prepared using a sandy loam soil which contained the crop species barley (Hordeum vulgare), spring wheat (Triticum aestivum), sorghum (Sorghum bicolor) and corn (Zea mays).

All treatments were maintained in the greenhouse for an additional 21 days at which time visual assessments of weed control were made using a scale of 0 to 100 for each species where 0 represented no control and 100 represented complete control. The results are shown in Table D.

TABLE D

| CMPD 3 | | | | | | |
|---|---|---|---|---|---|---|
| RATE = G/HA | 125 | 64 | 32 | 16 | 8 | 4 |
| PREEMERGENCE | | | | | | |
| WINTER WHEAT | 100 | 95 | 95 | 95 | 80 | 80 |
| KOCHIA | 95 | 90 | 90 | 80 | 80 | 60 |
| DOWNY BROME | 100 | 100 | 95 | 90 | 90 | 80 |
| GREEN FOXTAIL | 100 | 100 | 100 | 100 | 80 | 60 |
| SPRING WHEAT | 100 | 100 | 100 | 95 | 90 | 70 |
| WILD OATS | 100 | 95 | 90 | 70 | 60 | 30 |
| RYE | 100 | 90 | 80 | 75 | 50 | 20 |
| BARLEY | 100 | 95 | 95 | 95 | 90 | 80 |
| CORN | 80 | 75 | 70 | 70 | 50 | 50 |
| SORGHUM | 100 | 100 | 100 | 95 | 90 | 85 |
| FIELD BINDWEED | 90 | 90 | 80 | 70 | 60 | 30 |
| JOINT GOATGRASS | 90 | 90 | 90 | 90 | 50 | 40 |
| WILD BUCKWHEAT | 100 | 95 | 90 | 70 | 70 | 50 |
| LAMBSQUARTER | 100 | 100 | 100 | 95 | 70 | 40 |
| PIGWEED | 90 | 90 | 90 | 90 | 80 | 60 |

| CMPD 3 | | | | | | |
|---|---|---|---|---|---|---|
| RATE = G/HA | 125 | 64 | 32 | 16 | 8 | 4 |
| POSTEMERGENCE | | | | | | |
| KOCHIA | 100 | 100 | 100 | 100 | 100 | 65 |
| DOWNY BROME | 100 | 100 | 100 | 100 | 100 | 30 |
| GREEN FOXTAIL | 100 | 100 | 100 | 100 | 100 | 80 |
| WILD OATS | 100 | 100 | 100 | 100 | 95 | 70 |
| WINTER WHEAT | 100 | 100 | 100 | 100 | 95 | 60 |
| RYE | 100 | 100 | 100 | 80 | 80 | 50 |
| FIELD BINDWEED | 95 | 95 | 90 | 90 | 60 | 20 |

TABLE D

| CMPD 7 | | | | | | | |
|---|---|---|---|---|---|---|---|
| RATE = G/HA | 125 | 64 | 32 | 16 | 8 | 4 | 2 |
| PREEMERGENCE | | | | | | | |
| WINTER WHEAT | 100 | 80 | 70 | 70 | 60 | 60 | 40 |
| RUSSIAN THISTLE | 90 | 80 | 70 | 50 | 50 | 40 | 30 |
| KOCHIA | 100 | 100 | 100 | 100 | 100 | 70 | 50 |
| DOWNY BROME | 100 | 100 | 100 | 95 | 95 | 90 | 80 |
| GREEN FOXTAIL | 100 | 100 | 100 | 100 | 100 | 100 | 95 |
| SPRING WHEAT | 100 | 100 | 100 | 100 | 100 | 100 | 30 |
| WILD OATS | 100 | 95 | 90 | 80 | 50 | 40 | 20 |
| RYE | 95 | 90 | 80 | 70 | 65 | 50 | 35 |
| BARLEY | 100 | 100 | 100 | 100 | 90 | 75 | 40 |
| CORN | 100 | 100 | 100 | 95 | 90 | 80 | 50 |
| SORGHUM | 100 | 100 | 100 | 100 | 100 | 95 | 80 |
| FIELD BINDWEED | 80 | 80 | 80 | 70 | 40 | 30 | 10 |
| JOINT GOATGRASS | 100 | 100 | 90 | 80 | 80 | 70 | 60 |
| WILD BUCKWHEAT | 100 | 90 | 90 | 80 | 70 | 60 | 50 |
| LAMBSQUARTER | 100 | 100 | 100 | 90 | 80 | 80 | 60 |
| PIGWEED | 100 | 100 | 95 | 90 | 80 | 80 | 70 |

| CMPD 7 | | | | | | | |
|---|---|---|---|---|---|---|---|
| RATE = G/HA | 125 | 64 | 32 | 16 | 8 | 4 | 2 |
| POSTEMERGENCE | | | | | | | |
| RUSSIAN THISTLE | 100 | 100 | 100 | 98 | 60 | 20 | 0 |
| KOCHIA | 100 | 100 | 100 | 100 | 100 | 95 | 85 |
| DOWNY BROME | 100 | 100 | 100 | 100 | 100 | 90 | 75 |
| GREEN FOXTAIL | 100 | 100 | 100 | 100 | 100 | 100 | 100 |
| WILD OATS | 100 | 100 | 100 | 100 | 100 | 100 | 100 |
| WINTER WHEAT | 100 | 100 | 100 | 100 | 100 | 100 | 100 |
| RYE | 100 | 100 | 100 | 100 | 100 | 100 | 80 |
| FIELD BINDWEED | 100 | 100 | 100 | 100 | 100 | 70 | 50 |

Test E

Plastic windowsill flats were filled with planting medium and seeded with morningglory (Ipomea spp.), purple nutsedge (Cyperus rotundus), pigweed (Amaranthus spp.), bindweed (Convolvulus spp.), narrowleaf panicum (Panicum maximum), common ragweed (Ambrosia elatior), sandbur (Cenchrus echinatus), itchgrass (Rottboellia exaltata), johnsongrass (Sorghum halepense), guineagrass (P. maximum), smooth crabgrass (Digitaria ischaemum), broadleaf signalgrass (Brachiaria plantaginea), bermudagrass (Cynodon dactylon), goosegrass (Eleusine indica), large crabgrass (D. sanguinalis) and yellow nutsedge (C. esculentus).

The plantings were treated preemergence and postemergence with the compounds formulated in a non-phytotoxic spray solution. The plantings were staggered so that the preeemergence and postemergence treatments were sprayed on the same day. The postemergence treatments also included Kentucky bluegrass, sweet white clover, tall fescue and sugarcane. Treatments were visually rated 20 or 21 days after treatment and compared with the appropriate controls. The injury ratings were based on the scale of 0 to 100 where 0 indicates no effect, 20 indicates minimal effect and 100 indicates complete control. The results are shown in Table E.

TABLE E

| | CMPD 3 |
|---|---|
| RATE G/HA | 32 |
| PREEMERGENCE | |
| JOHNSONGRASS | 100 |
| GUINEA-GRASS | 0 |
| BRDLF SGNLGRASS | 60 |
| BERMUDAGRASS | 50 |
| GOOSEGRASS | 70 |
| LARGE CRABGRASS | 100 |
| YELLOW NUTSEDGE | 90 |
| SANDBUR | 60 |
| BINDWEED | 60 |
| PIGWEED | 100 |
| MORNINGLORY | 0 |
| CMN RAGWEED | 40 |
| PURPLE NUTSEDGE | 100 |
| SMOOTH CRABGRAS | 50 |
| ITCH-GRASS | 0 |
| N/LF PANICUM | 0 |

| | CMPD 3 |
|---|---|
| RATE G/HA | 32 |
| POSTEMERGENCE | |
| SUGARCANE | 40 |
| JOHNSONGRASS | 100 |
| GUINEA-GRASS | 100 |
| BRDLF SGNLGRASS | 100 |
| BERMUDAGRASS | 0 |
| GOOSEGRASS | 0 |
| LARGE CRABGRASS | 100 |
| YELLOW NUTSEDGE | 80 |
| SANDBUR | 100 |
| BINDWEED | 100 |
| PIGWEED | 100 |
| MORNINGLORY | 100 |
| CMN RAGWEED | 0 |
| PURPLE NUTSEDGE | 100 |
| KTY. BLUEGRASS | 90 |
| WHITE CLOVER | 100 |
| TALL FESCUE | 80 |
| SMOOTH CRABGRAS | 100 |
| ITCH-GRASS | 100 |
| N/LF PANICUM | 100 |

Test F

The object of this test was to evaluate the preemergence efficacy of Compound 3 and the postemergence safety of the compound to sugarcane and pineapple plants. The weeds and their plantings were as described in Test E.

Compound 3 was sprayed preemergence to the weeds and postemergence to sugarcane and pineapple, thereby simulating a type of field use of the compound. The compound was formulated in a non-phytotoxic solvent. Plants were visually rated 26 days after treatment and compared with the appropriate controls. The injury ratings were based on the scale of 0 to 100, where 0 indicates no effect, 20 indicates minimal effect and 100 indicates complete control. The results are shown in Table F. The variation in the results of this test from those of Test E could be due to the fact that the tests were conducted at different times and/or involved different growth stages of plants at time of treatment.

Table F

| Species | Compound 3 | |
|---|---|---|
| | 32 | 16 g/ha |
| | Preemergence | |
| Morningglory | 90 | 70 |
| Purple nutsedge | 100 | 100 |
| Pigweed | 100 | 90 |
| Bindweed | 90 | 60 |
| Narrowleaf panicum | 100 | 50 |
| Ragweed | 70 | 50 |
| Sandbur | 100 | 100 |
| Itchgrass | 80 | 30 |
| Johnsongrass | 100 | 100 |
| Guineagrass | 100 | 70 |
| Smooth crabgrass | 100 | 70 |
| Broadleaf signalgrass | 60 | 0 |
| Goosegrass | 100 | 30 |
| Large crabgrass | 100 | 70 |
| Yellow nutsedge | 90 | 60 |
| | Postemergence | |
| Sugarcane | 10 | 0 |
| Pineapple | 0 | 0 |

Test G

Budded citrus seedlings were planted in 30-liter plastic pots. The pots were also seeded with sandbur, guineagrass, narrowleaf panicum, common ragweed, yellow nutsedge, purple nutsedge and bindweed. Compound 3 was applied preemergence and postemergence to the weeds. The treatment was sprayed to simulate the trunk-to-trunk herbicide application method used in citrus groves. The compound was also applied postemergence to windowsill flats of weeds described in Test E.

In these treatments Compound 3 was formulated with 0.25% X-77 surfactant. Plants were visually rated 21 and 29 days after treatment and compared with the appropriate controls. The injury ratings were based on the scale of 0 to 100, where 0 indicates no effect, 20 indicates minimal effect and 100 indicates complete control. The results are shown in Table $G_a$ and $G_b$. The variations in these results from those observed with Tests E and F could be due to the fact that the tests were conducted at different times of the year.

Table Ga

| Species | Compound 3 Preemergence 32 g/ha | Compound 3 Postemergence 32 g/ha |
|---|---|---|
| Citrus | 0 | 0 |
| Sandbur | 70 | 100 |
| Guineagrass | 30 | 90 |
| Narrowleaf panicum | 40 | 50 |
| Common ragweed | 50 | 80 |
| Yellow nutsedge | 30 | 100 |
| Purple nutsedge | 40 | 100 |
| Bindweed | 80 | 100 |

Table Gb

| Species | Compound 3 Postemergence 32 g/ha |
|---|---|
| Morningglory | 90 |
| Purple nutsedge | 100 |
| Pigweed | 100 |
| Bindweed | 100 |
| Narrowleaf panicum | 70 |
| Common ragweed | 100 |
| Sandbur | 100 |
| Itchgrass | 100 |
| Johnsongrass | 100 |
| Guineagrass | 100 |
| Smooth crabgrass | 100 |
| Bermudagrass | 0 |
| Goosegrass | 60 |
| Large crabgrass | 100 |
| Yellow nutsedge | 90 |

Test H

The object of this test was to evaluate Compound 3 for postemergence weed control and safety to coffee and sour lemon. The sour lemon, coffee, lantana (Lantana camara) and trumpetcreeper (Campsis radicans) plants were grown in individual 11.4-cm square plastic pots. The lalang (Impereta cylindrica) was grown in 15.2-cm plastic pots and the plants were at boot stage at the time of treatment.

Plants were sprayed postemergence with Compound 3 in a non-phytotoxic solvent. Plants were visually rated 26 days after treatment and compared with the appropriate controls. The injury ratings were based on the scale of 0 to 100 where 0 indicates no effect, 20 indicates minimal effect and 100 indicates complete control. The results are shown in Table H.

Table H

| Species | Compound 3 | | |
|---|---|---|---|
| | Postemergence | | |
| | 32 | 16 | 8 g/ha |
| Sour lemon | 10 | 0 | 0 |
| Coffee | 10 | 0 | 0 |
| Lantana | 0 | 0 | 0 |
| Trumpetcreeper | 0 | 0 | 0 |
| Lalang | 90 | 90 | - |

## Claims

1. A method for controlling undesired vegetation in crops comprising maize (corn), soybeans and plantation crops and on fallow land said method comprising applying to the locus of the crop to the protected a herbicidally effective amount of a compound of Formula I

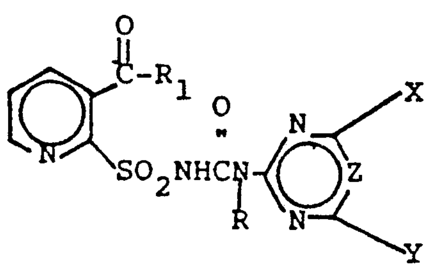

I

wherein
R is H or $CH_3$;
$R_1$ is $CH_3$, $CH_2CH_3$ or $(CH_2)_2CH_3$;
X is $CH_3$, $OCH_3$ or Cl;
Y is $CH_3$ or $OCH_3$; and
Z is CH or N.

2. The method of Claim 1 wherein the crop is a plantation crop.

3. The method of Claim 1 wherein
R is H;
$R_1$ is $CH_3$ or $CH_2CH_3$;
X is $OCH_3$;
Y is $CH_3$ or $OCH_3$; and
Z is CH.

4. A method of Claim 1 wherein the compound is N-[[(4-chloro-6-methoxypyrimidin-2-yl)amino]-carbonyl]-3-(1-oxobutyl)-2-pyridinesulfonamide.

5. The method of Claim 3 wherein the compound is N-[[(4,6-dimethoxypyrimidin-2-yl)amino]-carbonyl]-3-(1-oxoethyl)-2-pyridinesulfonamide.

6. The method of Claim 3 wherein the compound is N-[[(4,6-dimethoxypyrimidin-2-yl)amino]-carbonyl]-3-(1-oxopropyl)-2-pyridinesulfonamide.

7. The method of Claim 4 wherein the locus to be protected is a maize (corn) crop.

8. The method of Claim 5 wherein the locus to be protected is a plantation crop.

9.The method of Claim 5 wherein the locus to be protected is fallow land.

10. The method of Claim 6 wherein the locus to be protected is a plantation crop.
11. The method of Claim 10 wherein the plantation crop is sugar cane.
12. The method of Claim 10 wherein the plantation crop is pineapple.
13. The method of Claim 10 wherein the plantation crop is citrus.
14. The method of Claim 10 wherein the plantation crop is coffee.
15. The method of Claim 6 wherein the locus to be protected is fallow land.

European Patent Office

# EUROPEAN SEARCH REPORT

Application Number

EP 88 31 0907

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL PATENTS INDEX, Basic Abstracts Journal, 26th August 1987, Section C, week 8726, abstract no. 87-181902/26, Derwent Publications Ltd, London, GB; & JP-A-62 111 982 (ISHIHARA SANGYO KAISHA) 22-05-1987 (Cat. D) * Whole abstract * --- | 1-15 | A 01 N 47/36<br>C 07 D 401/12 |
| X | EP-A-0 245 058 (DU PONT DE NEMOURS) * Claim 16; page 20, lines 17-21; page 185, lines 20-30 * ----- | 1-15 | |
| | | | **TECHNICAL FIELDS SEARCHED (Int. Cl.4)** |
| | | | A 01 N<br>C 07 D |

The present search report has been drawn up for all claims

| Place of search | Date of completion of the search | Examiner |
|---|---|---|
| THE HAGUE | 02-03-1989 | DECORTE D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document